# EUROPEAN PATENT SPECIFICATION

(11) **EP 1 551 986 B1**
(45) Date of publication and mention of the grant of the patent: **27.08.2014**
(21) Application number: 03770601.7
(22) Date of filing: 30.09.2003
(51) Int. Cl.: C12P 19/34, C12N 15/11

(54) **POLYNUCLEOTIDE SYNTHESIS AND LABELING BY KINETIC SAMPLING LIGATION**
POLYNUKLEOTIDSYNTHESE UND MARKIERUNG DURCH LIGATION MIT KINETISCHEM SAMPLING
SYNTHESE ET MARQUAGE DE POLYNUCLEOTIDES PAR LIGATURE D'ECHANTILLONNAGE CINETIQUE

(30) Priority: 30.09.2002 US 415043 P; 07.02.2003 US 446184 P
(43) Date of publication of application: 13.07.2005
(73) Proprietor: Affymetrix, Inc., Santa Clara, CA 95051 (US)
(72) Inventor: NAMSARAEV, Eugeni, Sunnyvale, CA 94086 (US)
(74) Representative: Legg, James Christopher
(86) International application number: PCT/US2003/031059
(87) International publication number: WO 2004/029223

(56) References cited:
- EP-A1- 1 327 682
- WO-A2-02/06469
- WO-A2-2004/092375
- US-A- 5 494 810
- US-A- 5 525 470
- US-A- 5 696 249
- US-A- 5 858 731
- US-A1- 2002 051 986
- LEBEDENKO E N ET AL: "METHOD OF ARTIFICIAL DNA SPLICING BY DIRECTED LIGATION (SDL)" NUCLEIC ACIDS RESEARCH, OXFORD UNIVERSITY PRESS, SURREY, GB, vol. 19, no. 24, 1 January 1991 (1991-01-01), pages 6757-6761, XP002069446 ISSN: 0305-1048
- PRITCHARD CLARE E ET AL: "Effects of base mismatches on joining of short oligodeoxynucleotides by DNA ligases" NUCLEIC ACIDS RESEARCH, vol. 25, no. 17, 1997, pages 3403-3407, XP002582710 ISSN: 0305-1048
- CHALMERS FELICITY MEREDITH ET AL: "Scaling up the ligase chain reaction-based approach to gene synthesis" BIOTECHNIQUES, vol. 30, no. 2, February 2001 (2001-02), pages 249-252, XP001183953 ISSN: 0736-6205
- BARANY F: "GENETIC DISEASE DETECTION AND DNA AMPLIFICATION USING CLONED THERMOSTABLE LIGASE" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.88.1.189, vol. 88, no. 1, 1 January 1991 (1991-01-01), pages 189-193, XP000368693 ISSN: 0027-8424
- BORODINA TATIANA A ET AL: "Ligation-based synthesis of oligonucleotides with block structure." ANALYTICAL BIOCHEMISTRY, vol. 318, no. 2, 15 July 2003 (2003-07-15) , pages 309-313, XP002582712 ISSN: 0003-2697
- HARDENBOL PAUL ET AL: "Multiplexed genotyping with sequence-tagged molecular inversion probes" NATURE BIOTECHNOLOGY, NATURE PUBLISHING GROUP, NEW YORK, NY, US LNKD- DOI:10.1038/NBT821, vol. 21, no. 6, 1 June 2003 (2003-06-01), pages 673-678, XP002292164 ISSN: 1087-0156

## Description

### FIELD OF THE INVENTION

The present invention is in the field of molecular biology, and relates to the construction of polynucleotides suitable for use as genetic probes and other purposes. More specifically, the invention relates to methods for the construction of polynucleotides by ligation of shorter oligonucleotides without the need to first purify the oligonucleotides and without the need for a polynucleotide template. The invention also relates to methods of ligating oligonucleotides to one or both ends of a single-stranded polynucleotide.

### BACKGROUND OF THE INVENTION

A variety of modern molecular biology techniques call for relatively long single-stranded probes.

For example, in the genotyping methods described in WO 02/057491 and Hardenbol et al., Nature Biotechnol. 21(6): 673-678 (2003), each probe includes at least two targeting domains complementary to the nucleic acid target and at least one universal priming site, and may additionally include further universal priming sites, one or more genotypic labels ("bar code" sequences), and other sequences: the probes can thus readily exceed 80 nt in length. Various types of multiplex polymerase chain reactions ("PCR"), RNA profiling methods and genetic expression scoring methods similarly call for long single-stranded ("ss") probes.

In a variety of these methods, a plurality of long single-stranded probes are combined and used as a mixture, or library, in multiplex analyses.

Current methods for constructing long single-stranded polynucleotides are cumbersome and inefficient, particularly so for preparing libraries comprising a plurality of such probes.

Direct chemical synthesis of polynucleotides of the required length provides low overall yields and a high proportion of contaminating truncated oligonucleotides that lack one or more nucleotides at one or both of their termini. For example, even at 99% efficiency per coupling cycle, a synthesis of a 120-base oligonucleotide would be expected to yield only 3% full-length product, along with a 30-fold molar excess of truncates. The low yield necessitates a large scale synthesis in order to obtain enough of the final product, which is expensive, and the high proportion of truncates necessitates purification of the full-length product. In addition to their low yield, such chemical synthesis methods require the separate synthesis of each of the probes, which must thereafter be combined for multiplex analysis.

An alternative to direct chemical synthesis, PCR synthesis, is error-prone, even with proofreading polymerases having 3' to 5'exonuclease activity. Furthermore, amplification-based synthesis methods require a template polynucleotide that is complementary to the entire length of desired probe sequence. When probe-length template is unavailable from natural sources, the template typically must be synthesized chemically, recapitulating the problems and disadvantages that inhere in direct chemical synthesis of long oligonucleotides.

Another alternative to direct chemical synthesis, Template-Directed Ligation (TDL), involves ligation of a number of oligonucleotides that are aligned for ligation by hybridization to a polynucleotide template complementary to the entire length of the desired polynucleotide product. U.S. Pat. No. 5,998,175 describes a TDL-based method using pentameric oligonucleotides, which are short enough to be ligated without first being purified from truncated contaminants, since truncations comprise only a small fraction of synthesis products for such short oligonucleotides. As with PCR, however, the requirement for a separate template, with a different sequence for each member of a series of polynucleotide probes, makes TDL impractical for synthesis of long-stranded probes, particularly so for synthesis of an entire library of such probes.

Methods that require full-length templates also present the problem of isolating the probe from a template of identical or near-equivalent length.

Another ligation-based technique obviates the need for full-length template. In this method, two shorter substrate oligonucleotides are held in proper register for ligation by stable hybridization to a third oligonucleotide that is complementary to each of the substrate oligonucleotides at the ends to be ligated; the third oligonucleotide is of subtemplate length.

Although such methods obviate the need for a full-length template, they require that the substrate oligonucleotides be purified from truncates prior to ligation: truncated contaminants, particularly those lacking only one or two terminal nucleotides or a terminal phosphate group at the end to be ligated, can stably form unproductive complexes with the third oligonucleotide, severely inhibiting ligation of the full-length oligonucleotides to form the desired full-length polynucleotide product.

The need thus exists for an efficient, cost-effective method of constructing long single-stranded polynucleotides. The method should not require the prior construction of a separate full-length polynucleotide template for each desired probe. The method should permit use of oligonucleotides without their prior purification from truncated contaminant molecules. When possible, the method should exploit the fact that, in some analytical methods, the majority of the sequence in each probe molecule is the same, regardless of the genetic variant to be detected or locus to be analysed, with sequence variation limited to specific well-defined portions within the probes. The method should be amenable to use in multiplex format where the entire range of sequences in the library can be created at the same time, in the same synthesis reaction, rather than in separate reactions. The method should exhibit high ligation efficiency, resulting in a high yield of the desired polynucleotide probes.

### SUMMARY OF THE INVENTION

The present invention satisfies these and other needs in the art by providing a method for constructing a polynucleotide suitable for use as a genetic probe by ligating three shorter oligonucleotides. The invention provides a method of constructing at least one species of product polynucleotide using populations of truncate-containing oligonucleotide synthesis products, the method comprising:
ligating together a central oligonucleotide, at least one species of first oligonucleotide, and at least one species of second oligonucleotide,
the first, central and second oligonucleotides being annealed to a common scaffold oligonucleotide during ligation,
the scaffold oligonucleotide being complementary to the entire length of the central oligonucleotide, such that the duplex formed is stable under the ligation conditions, and the scaffold oligonucleotide being complementary to the first and second oligonucleotides only over a limited number of nucleotides, such that the duplexes formed are not stable under the ligation conditions, and wherein said scaffold oligonucleotide is subtemplate in length.
A scaffold oligonucleotide is provided that is complementary or partly complementary to all three oligonucleotides to be ligated. There is no requirement for a full-length template strand, *i.e.* the scaffold oligonucleotide is subtemplate in length. The scaffold oligonucleotide serves to properly align the three oligonucleotides for ligation, but does not extend the entire length of the desired ligation product. The ligation conditions are chosen so that annealing of outer oligonucleotides to the scaffold is unstable. Such conditions render the ligation reaction resistant to inhibition by nonligatable contaminants in oligonucleotide preparations, meaning that unpurified oligonucleotides can be used.

In one embodiment, a first oligo, a central oligo and a second oligo to be ligated are annealed to a scaffold oligonucleotide that is complementary to the entire length of the central oligo but only complementary at its (the scaffold oligonucleotide's) ends to a short region at one end of each of the first and second oligos. The scaffold and central oligos are purified away from truncated and other ligation-defective forms present in the mixture of products from their synthesis reactions. The first and second oligos are used without purification, as the entire population of their respective oligonucleotide synthesis reactions, including truncates and other ligation-defective contaminants.

The ligation conditions, and the lengths of the regions of complementarity between the oligonucleotides, are chosen such that during ligation the scaffold is unstably annealed to the first and second oligos. Such unstable annealing represents a way of sampling the populations of truncate-containing synthesis products for the first and second oligonucleotides, in that duplexes form and dissociate rapidly under such conditions. Any duplexes formed with contaminating ligation-defective oligos will not be good substrates for ligation and will dissociate rapidly. Eventually the desired full-length synthesis products in the first and second oligonucleotide synthesis reaction mixtures will form duplexes with the scaffold and be ligated to the central oligonucleotide. Such ligation is a sampling process in that the ligase enzyme is able to selectively ligate the desired full-length synthesis products despite the presence of an excess of contaminants. A ligation reaction performed under such conditions may be referred to as a "sampling ligation."

A ligase enzyme and appropriate cofactors, salts and buffers are added, and the reactions are incubated at an appropriate temperature until ligation is substantially complete. Optionally, the full-length ligation product may then be separated from the scaffold oligonucleotide and any other unligated oligonucleotides by denaturation and size-based separation, such as denaturing polyacrylamide gel electrophoresis (PAGE) or column chromatography. The desired full-length ligation product, constructed by ligation of the first, central and second oligonucleotides, is referred to as the product polynucleotide.

The method may be performed by sequential addition of oligonucleotides or it may be performed in a single step. The regions of complementarity between the scaffold and the first or second oligos may vary, e.g. from 5 to 10 nt long, and ligation may be performed at various temperatures, e.g. from 20°C to 60°C. The only requirement for unstable annealing between the scaffold and the first and second oligos is that the ligation temperature be high enough to prevent stable duplex formation. The method can be used with the products of a single oligonucleotide synthesis reaction for each of the first and second oligos, or may be used with a plurality of oligonucleotide syntheses, the full-length products of which differ in sequence from each other. The products of five to 100 separate oligonucleotide syntheses may be pooled to form the populations of first or second oligonucleotides to be sampled.

The method of the present invention may also be used to extend a target single-stranded polynucleotide by ligating an oligonucleotide to the target polynucleotide's 3' end, its 5' end, or both. Separate scaffold oligonucleotides are used for the 3' and 5' end, making it possible to append different extending oligonucleotides at the opposing ends of the target polynucleotide. Ligation may be performed under conditions in which the scaffold oligonucleotide is unstably annealed to the target polynucleotide.

The oligonucleotides appended to the target polynucleotide may contain sequences useful in subsequent experimental manipulations, such as PCR primer binding sites, bar code sequences, or type IIS restriction endonuclease binding sites.

The method may be also used to label either the 5' or 3' end of an oligonucleotide, or a plurality of oligonucleotides, for example by adding a radioactive or fluorescent label.

The method of the present invention may also be used to ligate two or more species of oligonucleotides in the presence of a up to a ten-fold molar excess of truncate forms of one or more of the species with less than a three-fold decrease in ligation efficiency when compared with the same ligation reaction performed without truncate forms.

### BRIEF DESCRIPTION OF THE FIGURES

These and other objects and advantages of the present invention will be apparent upon consideration of the following detailed description, taken in conjunction with the accompanying drawings, in which like graphical representations refer to like structures throughout, and in which:

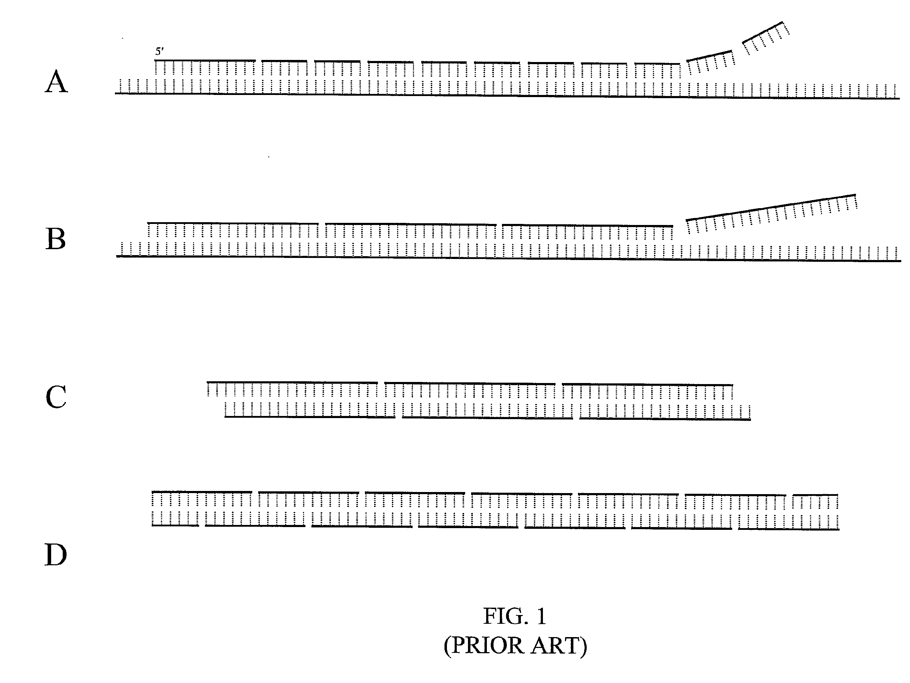

FIG. 1 is a schematic representation of several previous methods used to construct polynucleotides. FIG. 1A shows the method of U.S. Pat. No. 5,998,175; FIG. 1B shows the method of U.S. Pat. No. 6,110,668; FIG. 1C shows the method of U.S. Pat. No. 5,380,831; and FIG. 1D shows the method of Chalmers et al., 2001, BioTechniques 30: 249. In this and all schematic representations herein, pairs of vertical dashed lines aligned co-linearly are intended to indicate base pairing generally, and the number of dashed lines is not intended to reflect any specific number of basepairs. The schematics also are not intended to accurately reflect the relative lengths of the oligonucleotides or polynucleotides depicted. In all schematics, unless otherwise indicated, the left end of the upper strands represents the 5' end, and the right end represents the 3' end. The directionality of the bottom strands is reversed, with left ends representing 3' ends and right ends representing 5' ends.
FIG. 2A is a schematic representation of a ligation method according to the present invention.
FIG. 2B is a schematic representation of the principle of kinetic sampling ligation. Despite this schematic, the present invention is not limited by theory.
FIG. 3 is a schematic of a pair of ligation reactions designed to test the effect of contaminating nonligatable oligonucleotides on full-length product formation in kinetic sampling ligation reactions. The number of oligonucleotide molecules depicted is not intended to accurately reflect their molar ratio in the reaction.
FIG. 4A is a schematic of a method of extending both the 3' and 5' ends of a single-stranded polynucleotide according to the present invention.
FIG. 4B is a schematic of the preparation of single stranded fragments from genomic DNA for random cloning. The fragments produced are suitable for extension according to the method illustrated in FIG. 4A.
FIGS. 5A and 5B are schematics of methods of labeling the 3' end of an oligonucleotide according to the present invention. The label is depicted as attached to the 3' end of the labeling oligonucleotide, but may be attached at other locations on the labeling oligonucleotide provided that it does not interfere with the ligation reaction.
FIG. 6 shows a polyacrylamide gel of the products of ligation reactions performed according to the present invention.
FIG. 7 shows densitometric scans of the lanes of an electrophoresis gel demonstrating the formation of adenylated oligonucleotides during ligation reactions.
FIG. 8 shows a polyacrylamide gel of the products of a labeling reaction performed according to the present invention.
FIG. 9 is a plot showing the results of a series of multiplex labeling reactions performed according to the present invention.
FIG. 10 is a plot comparing the results of multiplex labeling reactions to singleplex labeling of the same oligonucleotides, each performed according to the present invention.

### DETAILED DESCRIPTION

Several prior methods of constructing polynucleotides are schematically illustrated in FIG. 1. These prior methods are inefficient for producing entire libraries of different species of polynucleotides suitable for use as genetic probes. FIG. 1A illustrates an embodiment of the method of U.S. Pat. No. 5,998,175, and FIG. 1B illustrates an embodiment of the method of U.S. Pat. No. 6,110,668. These two methods require a full-length template strand upon which the complementary strand, *i.e.* the polynucleotide product, is constructed. FIG. 1C illustrates an embodiment of the method of U.S. Pat. No. 5,380,831, and FIG. 1D illustrates an embodiment of the method of Chalmers et al., 2001, Bio Techniques 30: 249. These latter two methods entail synthesis of a full-length template along with the desired polynucleotide product. All four methods require that the template strand be separated from the polynucleotide product and discarded before the polynucleotide product can be used as a single-stranded genetic probe.

The methods illustrated in FIGS. 1A -1D also require that the oligonucleotides used to construct the polynucleotide product be purified prior to use, or that only very short oligonucleotides be used. Such purification is time-consuming and expensive. Methods requiring full-length templates and purification of oligonucleotides prior to ligation are ill-suited to construction of a library of a number of different species of polynucleotide for use as genetic probes, each of which is needed only in relatively small quantities.

The present invention, in one aspect, provides a method for constructing a library of polynucleotides suitable for use as probes in genetic screening. The method involves kinetic sampling ligation of three shorter oligonucleotides, two of which may be unpurified, into a product polynucleotide suitable for use as a genetic probe.

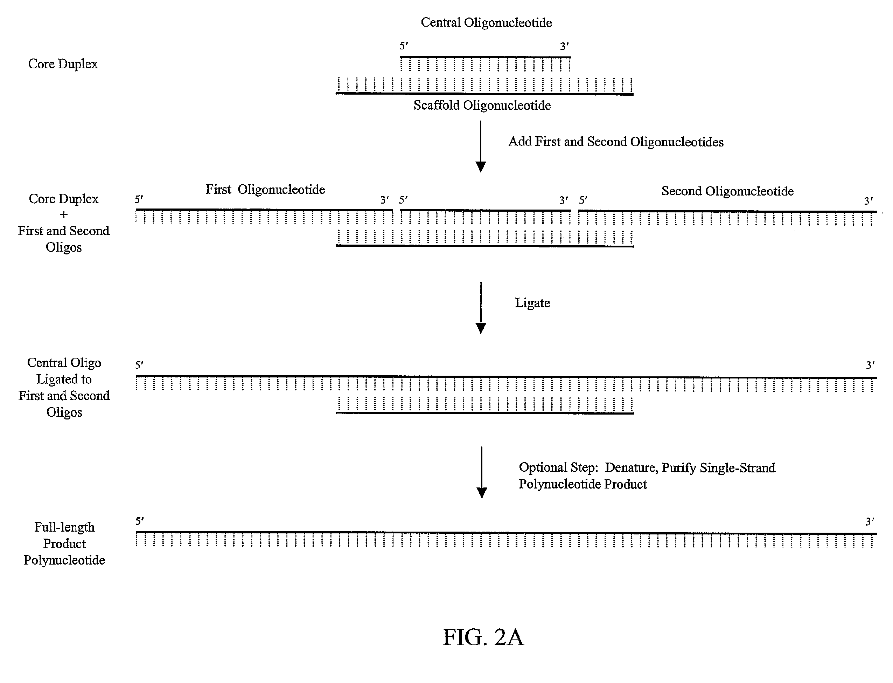

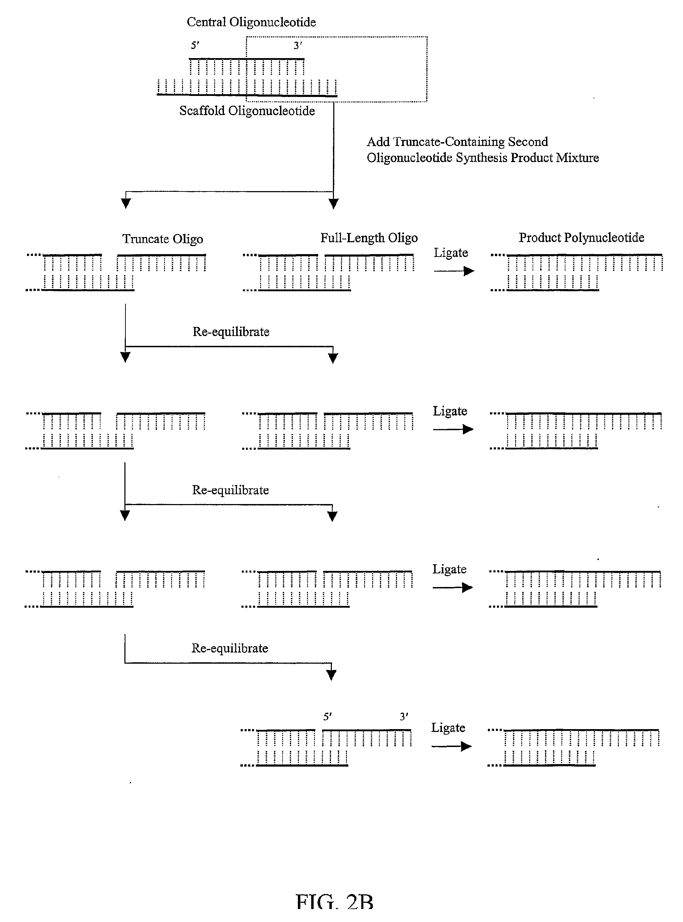

FIGS. 2A and 2B illustrate two aspects of the method. Referring to FIG. 2A, first and second oligos are ligated to the 5' and 3' termini, respectively, of a central oligonucleotide. Ligation of the three oligonucleotides is performed in the presence of an oligonucleotide scaffold complementary to the entire length of the central oligonucleotide and extending beyond both the 5' and the 3' ends of the central oligonucleotide.

The duplex formed by annealing the central and scaffold oligonucleotides is referred to as the core duplex. Core duplex may be formed by partially duplexing the central oligonucleotide with the scaffold oligonucleotide prior to contacting (e.g. mixing) the central and scaffold oligonucleotides with the first and second oligonucleotides. (The terms contacting, mixing and combining are used synonymously herein.) Alternatively, core duplex may be formed in the presence of the first and second oligonucleotides.

The bases of the oligonucleotide scaffold extending beyond the 5' end of the central oligonucleotide are complementary to the first oligo, and those extending beyond the 3' end of the central oligonucleotide are complementary to the second oligo. In the embodiment illustrated, when all three of the oligonucleotides to be ligated are base-paired to the scaffold oligonucleotide, no bases on the scaffold remain unpaired, and the ends of the first and second oligos are properly positioned for efficient ligation to the 5' and 3' ends, respectively, of the central oligonucleotide.

Oligonucleotides used in the ligation will typically be obtained by conventional solid-phase automated chemical synthesis, such as phosphoramidite synthesis, although oligonucleotides obtained in other ways may also be used. The method permits use of unpurified first and second oligonucleotides, *i.e*., crude populations of oligonucleotide synthesis reaction products where there has been no attempt to separate and remove truncated or otherwise defective oligonucleotides from the desired full-length oligonucleotide synthesis product after the oligonucleotides have been cleaved from the solid support. Such mixtures of chemical synthesis reaction products are herein referred to as unpurified regardless of any steps, such as deprotection, washing and rinsing, that are performed prior to the cleavage from the solid support. Reaction products are also referred to as unpurified despite steps subsequent to cleavage from the solid support, such as desalting or washing, so long as such steps are not designed to remove truncated synthesis products.

As used herein, the terms "oligonucleotide" and "polynucleotide" are not intended to reflect any particular number of nucleotides, and are used synonymously; both "oligonucleotide" and "polynucleotide" may refer to molecules of any length. The term "oligo," as used herein, is synonymous with "oligonucleotide."

In most applications, the scaffold oligonucleotide will be chemically synthesized, and will be no longer than necessary to provide the desired complementarity with other oligonucleotides, as illustrated in FIG. 2A. In other embodiments, however, single-stranded oligonucleotides obtained from natural sources may be used as the scaffold oligonucleotide. Such scaffold oligonucleotides may comprise additional nucleotides at their 3' end, 5' end, or both. These additional nucleotides extend beyond the region of complementarity of the scaffold oligonucleotide to the first and second oligonucleotides, are not complementary to the sequence of the desired product of the ligation reaction, and play no role in the synthesis of the polynucleotide product.

A mixture of core duplex, first and second oligos, ligase, salts, buffers and cofactors is then incubated under conditions wherein the first oligo and the second oligo do not form stable duplexes with the scaffold oligonucleotide. In one embodiment, the central oligonucleotide and the scaffold oligonucleotide form a stable core duplex under these conditions. Whether the core duplex is stable or unstable under ligation conditions, however, is not an essential aspect of the present invention.

Ligation under conditions that do not permit stable duplex formation between the core duplex and the first and second oligos is referred to herein as kinetic sampling ligation or simply sampling ligation, and is illustrated in FIG. 2B. For simplicity, sampling ligation is illustrated only for ligation of the second oligo, with the dotted box representing the relevant portion of the core duplex. An analogous process takes place with the first oligo at the other end of the core duplex (not shown).

As shown, an unpurified mixture of synthesis products for the second oligo, including truncates, is added to the core duplex, leading to complexes between core duplex and either the truncate or the full-length second oligo. The truncate complexes contain a gap, preventing them from being ligated, whereas the full-length complexes do not. Although only one truncate species, missing a single base, is illustrated in FIG. 2B, an unpurified mixture of oligonucleotide synthesis products may also contain truncate species missing two or more bases. Only full-length second oligonucleotides can be ligated to form the desired product polynucleotide. The unligated core duplex remaining in complexes with truncate oligonucleotides then re-equilibrates with the remaining truncate and full-length second oligos, again generating a mixture of truncate and full-length oligo complexes, as shown in the first re-equilibration step in FIG. 2B. Again, only complexes containing full-length second oligo can be ligated to form product polynucleotide.

After a number of cycles of such ligation and re-equilibration, all or a substantial percentage of the core duplex is ligated to full-length second oligo to form the desired polynucleotide product, provided that an excess of full-length second oligo over core duplex is present. Although only three such re-equilibration steps are shown in FIG. 2B, the actual number of such steps is dependent on a number of experimental variables, including but not limited to the proportion of truncate contaminants in the oligonucleotide synthesis product mixture.

The ligation reaction is allowed to proceed until the desired yield of full-length polynucleotide product is obtained, but typically no longer than is conveniently performed, typically hours or less. In terms of FIG. 2B, the reaction is allowed to proceed until enough cycles of ligation and re-equilibration take place to obtain the desired yield of polynucleotide product. The reaction conditions are selected to ensure that re-equilibration is sufficiently rapid that ligation reactions can be completed within a convenient period. The degree of completion of the reaction can be readily assessed by qualitative determination of the amount of full-length product polynucleotide produced, e.g., by gel electrophoresis.

Ligation conditions include the temperature, salt concentration, ionic strength, presence or absence of molecular crowding agents, pH and all other factors that influence the stability of nucleic acid base pairing. The length of duplex formed between the first or second oligo and the scaffold is another variable influencing the stability of nucleic acid duplexes, and thus the rate of re-equilibration of the core duplex with truncate and full-length first and second oligos. Duplexes that re-equilibrate rapidly relative to the timeframe of the ligation reaction are referred to as unstable duplexes.

The ligation of unstably duplexed oligos has significant advantages when ligating a mixture of oligonucleotides containing truncated forms of the full-length oligonucleotide. Such truncated forms are invariably created in the chemical synthesis of oligonucleotides, with truncates comprising a greater percentage of the total reaction products as the length of the oligonucleotide being synthesized increases. In prior methods, in which the presence of truncates inhibits the desired reaction, oligonucleotides over approximately twenty nucleotides must typically be purified away from contaminating truncates before use. Purification of such oligonucleotides is typically accomplished by preparative scale denaturing polyacrylamide gel electrophoresis, which is time and labor intensive, and thus expensive.

In the methods of the present invention, unstable duplexes formed by unligatable contaminants in the populations of first and second oligonucleotide synthesis products will dissociate relatively rapidly with respect to the time of the ligation reaction (*i.e*., re-equilibration will take place), and thus will not permanently block duplex formation by the full-length first or second oligo. Duplexes formed by the full-length first or second oligos will also be short-lived, but since ligation is irreversible, even relatively infrequent ligation of such transient "unstable" duplexes will eventually lead to the ligation of most, if not all, of the nucleic acid strands capable of being ligated, as illustrated in FIG. 2B.

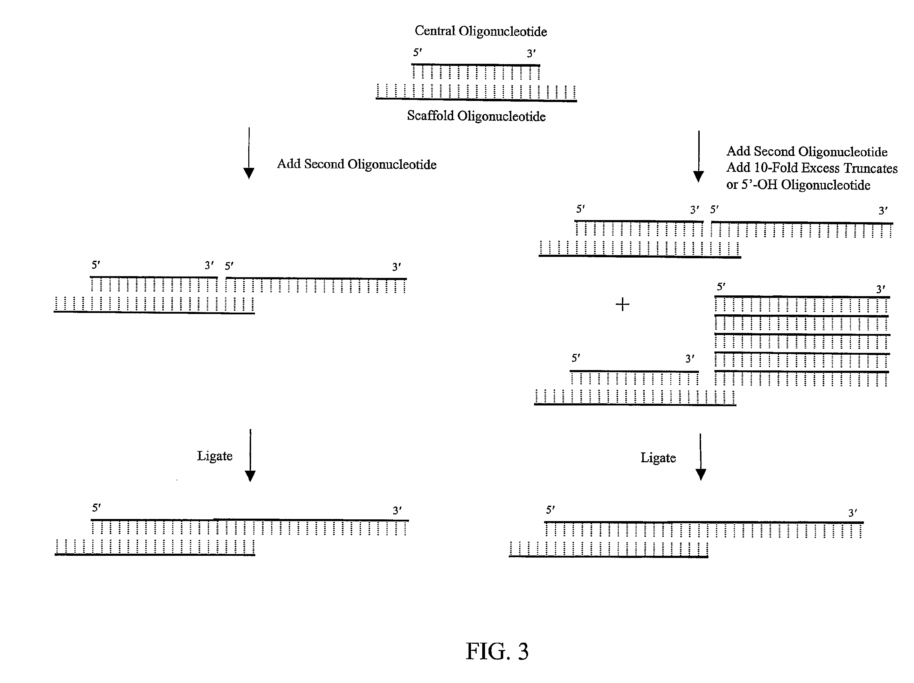

The sampling ligation reaction according to the present invention is dynamic: many short-lived duplexes between the core duplex and the first and second oligos form and dissociate during the course of the ligation reaction. It is this dynamic re-equilibration that prevents contaminating truncated oligos from forming long-lived duplexes, with half-lives comparable to the length of the ligation reaction, that would prevent the full-length oligo from binding. In this way, kinetic sampling ligation prevents truncated contaminants from inhibiting formation of the desired full-length ligation product. Example 1 herein below demonstrates the construction of a polynucleotide suitable for use as a genetic probe using one embodiment of a sampling ligation method of the present invention. An experimental method to determine whether duplex formation is "stable" or "unstable" is discussed below, illustrated in FIG. 3, and demonstrated in Example 3.

Unlike traditional ligation under stable duplex conditions, the presence of truncates does not prevent formation of the desired ligation product in the present invention. For this reason, it is possible with the current invention to use populations of truncate-containing oligonucleotide synthesis products as the first and second oligos. Example 3 illustrates this feature of the invention.

In one embodiment, ligation reaction conditions used in the methods of the present invention allow stable core duplex formation, and the temperature is low enough to be permissive for ligase activity. In another embodiment, the number of complementary bases between the central and scaffold oligonucleotides in the core duplex is greater than the number of complementary bases between the core duplex and either of the first or second oligos, such that a ligation temperature exists where the core duplex is stable but the complexes with the first and second oligos are unstable.

In one embodiment of the invention, the central oligonucleotide is annealed to the oligonucleotide scaffold to form the core duplex in a first step prior to the addition of the first and second oligonucleotides. The core duplex so formed may be stored for subsequent use in several different synthesis reactions.

Annealing, like hybridization, refers to the process of forming base pairs between complementary strands of nucleic acids. The annealed strands may be referred to as a duplex, and the process of creating a duplex may be referred to as duplexing. When fewer than all the bases of one or both strands are base paired the result is a partial duplex, and the process of creating a partial duplex may be referred to as partial duplexing. Annealing, base pairing, hybridizing and duplexing are used synonymously herein, as are anneal, basepair, hybridize and duplex. Annealing, base pairing, hybridizing and duplexing may be either stable or unstable. Unless otherwise indicated, complementary nucleic acid strands are assumed to form antiparallel, rather than parallel, duplexes.

Formation of a common core duplex is particularly advantageous when a library of genetic probes can be designed to incorporate all sequence differences into the first and second oligos, so that the central and scaffold oligonucleotides are identical for all members of the probe library. All members of the library of probes can then be constructed using the same core duplex by simply changing the first and second oligos that are used. In another embodiment, a single species of central oligonucleotide may be annealed to a mixture of different scaffold oligonucleotides to form a family of core duplexes differing only in the single-stranded "sticky end" regions of the scaffold.

The first oligo, the second oligo, or both, are then added to the core duplex. In one embodiment, both first and second oligos are present in at least a 5-fold, 6-fold, 7-fold, 8-fold, 9-fold, even at least 10-fold molar excess over the central oligonucleotide in the ligation.

Other embodiments of the invention involve the simultaneous addition of the first oligo, the second oligo, or both, along with the central and scaffold oligonucleotides. The order of addition of the first, second, central and scaffold oligonucleotide, however, is not an essential aspect of the present invention, and any order of addition falls within the scope of the invention.

A ligase is then added, along with any salts, buffers and cofactors required by the ligase. Other embodiments involve addition of ligase, salts, buffers and cofactors at different points in the reaction, and the timing of the addition is not an essential aspect of the invention. The ligation may be effected by use of a ligase enzyme such as T4 DNA ligase, T7 DNA ligase, Taq DNA ligase, *E*. *coli* DNA ligase, or thermostable Pfu DNA ligase, or any enzyme from any other organism, or any synthetic enzyme, that is capable of joining oligonucleotides together. RNA ligases may also be used if the oligonucleotides to be ligated are substrates for RNA ligase. The temperature of the ligation can be at least 20°C, 25°C, 30°C, 35°C, 42°C, 50°C or 60°C.

As mentioned above, relatively large quantities of core duplex comprising the central and scaffold oligonucleotides can be prepared in bulk and stored for use in several different reactions, and may be stored and aliquots thereof used many times. The central and scaffold oligonucleotides can be purified away from truncates prior to forming the core duplex. Since the central and scaffold oligonucleotides, in one embodiment of the present invention, are common to all members of a library of genetic probes, these two oligonucleotides can be purified with minimal expenditure of time and effort. For example, a library of 50 genetic probes would require 50 different species of first oligo and 50 different species of second oligo, but only one species of central and one species of scaffold oligonucleotide. Of the 102 different oligonucleotides required, only two would need to be purified. This represents a 98% savings in purification effort compared with prior methods that require purification of every oligonucleotide to prevent complications due to the presence of truncates.

After ligation of the first, central and second oligonucleotides to form the full-length product polynucleotide, the scaffold oligonucleotide and other unligated oligonucleotides can be separated from the product polynucleotide, or not, depending on the intended subsequent use of the product polynucleotide. In one embodiment, size-based separation, such as denaturing PAGE, can be used to remove the scaffold and other oligonucleotides. The scaffold oligonucleotide is significantly shorter than the full-length product polynucleotide and is thus easily separated. Separation can also be effected by liquid chromatography under denaturing conditions. The central oligonucleotide can be fluorescently labeled to allow easy detection of the full-length product during purification.

The present invention has numerous advantages when compared to prior methods of constructing polynucleotides, which are susceptible to interference by contaminating truncates present in the oligonucleotides being ligated. Many prior oligonucleotide synthesis methods rely on relatively low temperatures or extended regions of complementarity to ensure stable duplex formation during ligation. For example, U.S. Pat. No. 5,380,831, illustrated in FIG. 1C, performs ligation of 4-5 base pair duplexes at 15°C, where such duplexes are relatively stable, rather than 37 to 42°C. U.S. Patent No. 6,110,668, illustrated in FIG. 1B, discloses a method of ligating 80 to 130 nucleotide long oligonucleotides at 52°C. All 80 to 130 bases of these oligonucleotides are base paired during ligation so that even at 52°C the duplex is stable.

Prior ligation methods employed stable duplex conditions because it was expected that ligation would be more efficient if the complexes that act as substrates for ligation were long-lived. Short-lived complexes would have been expected to ligate only inefficiently, so the unstable ligation conditions used in the present invention might have been expected to give little or no ligation product. Surprisingly, not only does unstable sampling ligation according to the present invention result in successful ligation, it has the unexpected advantage of preventing truncates from interfering with ligation of the desired full-length first and second oligos.

The invention also does not require the availability, or the synthesis, of a full-length template, as do many prior methods. A template is a single-stranded polynucleotide that is complementary to the desired product strand along substantially the entire length of that product strand, and is therefore approximately the same length as the desired product. FIGS. 1A -1D illustrate the role of a full-length template in several such prior methods of polynucleotide construction.

The scaffold oligonucleotide of the present invention is of subtemplate length: it does not extend along substantially the entire length of the desired polynucleotide product. The scaffold oligonucleotide is complementary to the full length of the central oligonucleotide but generally extends only modestly beyond the ends of the central oligonucleotide. Such single-stranded sticky ends will typically be short enough that duplexes with the first and second oligos can be made unstable under convenient laboratory conditions. Embodiments of the invention use single-stranded regions at the ends of the scaffold oligonucleotide of 5, 6, 7, 8, 9, or 10 nucleotides. (Note that in some embodiments of the present invention, as discussed *supra,* additional nucleotides extend from one or both ends of the scaffold oligonucleotide that are not complementary to the desired full-length ligation product. Such scaffold oligonucleotides are nonetheless also referred to as "subtemplate" in length since the region relevant to the kinetic sampling ligation reaction, *i.e*. that region complementary to other oligos, does not extend substantially the entire length of the desired polynucleotide product. Such additional sequences at the ends of the scaffold oligonucleotide are irrelevant to the use of the oligonucleotide as a scaffold, provided that such sequences do not interfere with annealing of the oligonucleotides involved in the kinetic sampling ligation reaction.)

In one series of embodiments of the methods of the present invention, entire libraries of oligonucleotides are synthesized simultaneously using a multiplex ligation. A common core duplex is used to create all members of the library of probes; accordingly, all sequence variations among members of the library are localized to the portions of the first and/or second oligos extending beyond the scaffold oligonucleotide. The first and second oligos, and thus the full-length product polynucleotide, may extend beyond the scaffold oligonucleotide at either end, or at both ends, by 10, 15, 20, 25, 30, 50, 75 or more nucleotides.

In some embodiments, any number of different first oligo species can be ligated to the central oligonucleotide in the presence of a single predetermined second oligo species, or any number of different second oligo species can be ligated to the central oligonucleotide in the presence of a single predetermined first oligo species. The resulting reaction products will comprise a product polynucleotide with a defined sequence at one end and with a number of sequence variants at the other end.

For example, it is possible to perform multiplex ligation to create probes specific for several different genetic loci in the same ligation reaction. A first oligo species and a second oligo species are synthesized for each genetic locus to be analyzed. The first and/or second oligonucleotide can optionally include further sequences, such as one or more genotypic labels, commonly referred to as bar code tags. A bar code tag is a short sequence designed algorithmically to maximize discrimination on a microarray having complements of the respective tags; a 1:1 correspondence as between tag sequence and first and/or second oligonucleotide permits each oligonucleotide so labeled to be detected by detection of the bar code uniquely associated therewith. *See, e.g.,* Shoemaker et al., Nature Genet. 14(4):450-6 (1996); EP 0799897; Fan et al., Genome Res. 10:853-60 (2000); and U.S. Patent No. 6,150,516. The first and/or second oligonucleotide can additionally and optionally include one or more amplification primer binding sites.

All first and second oligos also comprise terminal regions complementary to regions at the 3' and 5' ends, respectively, of the scaffold oligonucleotide. All first and second oligos are ligated to the common core duplex in the same reaction mixture. The resulting mixture of products will contain full-length genetic probes for each genetic locus for which first and second oligos are supplied. This embodiment is particularly well suited for simultaneous construction of probes for only a relatively modest number of genetic loci.

In yet another embodiment, different scaffold oligonucleotides, rather than a common core duplex, are used. A common central oligonucleotide is annealed to a mixture of several different scaffold oligonucleotides, each of which is perfectly complementary to the entire length of the central oligonucleotide, but each of which differs from every other scaffold oligonucleotide at the single-stranded regions extending beyond the central oligonucleotide. A different scaffold oligonucleotide is included for each polynucleotide probe desired to be synthesized. The result is a mixture of different core duplexes.

Several different pairs of first and second oligos can then be added, such that there exists in the final ligation mixture exactly one first oligo species and exactly one second oligo species for each species of scaffold oligonucleotide. After ligation there will be one full-length product polynucleotide for each scaffold oligonucleotide added. In embodiments in which the polynucleotide is designed to probe genetic loci, there will be one full-length probe species for each genetic locus of interest. This embodiment is typically preferred for simultaneous construction of probes for a large number of genetic loci.

Alternatively, several variants of both the first and the second oligos for a given target genetic locus can be used.

Since first and second oligos are used without purification in a number of embodiments of the present invention, it is actually a mixture of the products of an oligonucleotide synthesis reaction that is used, rather than a single molecular species. The terms first oligo and second oligo as used herein include such mixtures of products. A mixture of multiple species of first or second oligo comprises mixtures of synthesis reaction products for each species synthesized. The number of such mixtures of products of oligonucleotide syntheses that can be used in multiplex ligation reactions in various embodiments of the present invention ranges from one to 2, 5, 10, 25, 50, 75, 100 or more.

In yet another embodiment, a plurality of first oligo species and a single second oligo species, or a plurality of second oligo species and a single first oligo species, are included for each of the multiple scaffold oligonucleotides. In embodiments in which the first and/or second oligonucleotide includes a region complementary to a genetic locus, the resulting mixture of products will comprise a library of probes for each locus of interest, such libraries having a single defined sequence at one end and a variety of sequences at the other end.

All of the various species of full-length product polynucleotides of a multiplex ligation can be purified from shorter contaminants and unreacted oligonucleotides by denaturing PAGE or agarose gel electrophoresis, provided that the lengths of all first and second oligos are selected to give approximately the same length full-length product. Denaturing PAGE or denaturing agarose gel electrophoresis will effectively resolve all of the full-length products from shorter contaminants and unreacted oligonucleotides, despite the sequence differences among the various products. Purification of the desired full-length probe, however, is not essential to the invention.

Whether a duplex formed by any particular pair of oligonucleotides is stable or unstable under any given set of ligation reaction conditions can be assessed by a ligation challenge experiment. The ligation challenge may be understood with reference to FIG. 3. Results of such a ligation challenge are presented in Example 3.

To perform a ligation challenge reaction, a core duplex is first formed by annealing a central oligonucleotide with a scaffold oligonucleotide. Two ligation reactions are then performed, one with addition of just the second oligo and the other with addition of the second oligo and a ten-fold molar excess of nonligatable competitor oligo. FIG. 3 illustrates addition of a truncate form of the second oligo, missing one nucleotide from the 5' end, but an oligonucleotide lacking a 5' phosphate group (*i.e*., a 5'-OH form) may also be used. FIG. 3 only shows five unligatable competitors for one full-length second oligonucleotide but is intended to represent a ten-fold excess. Ligation is performed under the specific ligation conditions to be tested, e.g. 30 minutes at 37°C - 42°C using T4 DNA ligase. The resulting ligation products are then analyzed by denaturing PAGE. If the presence of a ten-fold molar excess of truncates (or 5' OH oligonucleotides) decreases the amount of ligation product (comprising the central and second oligos) that is formed more than a factor of three, the ligation conditions support stable duplex formation between the second oligo and the scaffold. If the amount of ligation product formed is decreased by a factor of three or less in the presence of a ten-fold molar excess of truncates (or 5' OH oligonucleotides), the ligation conditions support unstable duplex formation between the second oligo and the scaffold.

An analogous ligation challenge (not shown) can be performed for the first oligonucleotide by adding a ten-fold excess of truncates missing one nucleotide at the 3' end of the first oligonucleotide.

Since the predominant contaminants in unpurified oligonucleotides are truncates, a showing that unstable duplexes are formed, as assessed by the ligation challenge test described here, suggests that such contaminants in unpurified oligonucleotide preparations will not significantly inhibit formation of the desired ligation product under the ligation conditions tested. Under such conditions it is therefore unnecessary to purify the oligonucleotides to be ligated, with consequent savings in time, effort and expense.

The advantages of sampling ligation, i.e. ligation under conditions where first and second oligos are unstably duplexed to the scaffold, are demonstrated in Example 3. The example compares the ligation efficiency for first and second oligos with 7 or 18 bases at their ends complementary to the scaffold. Under the conditions of the reaction (37°C and 42°C) a 7 basepair duplex would be expected to be far less stable than an 18 basepair duplex. As shown in Table 1, *infra,* ligation of oligonucleotides with 18 complementary bases is inhibited by contaminating truncates under conditions where ligation of oligonucleotides with only 7 complementary bases is not significantly inhibited. This insensitivity to the presence of competing contaminants is a major advantage of ligation under unstable duplex conditions.

There is described herein a kinetic sampling ligation method to extend a single-stranded polynucleotide by ligating an oligonucleotide to the 3' end, the 5' end, or both.

There are also described herein kits to perform such extension reactions.

The portions of the single-stranded polynucleotide adjacent to and comprising the 3' and 5' ends are referred to as the 3' and 5' terminal regions of the single-stranded polynucleotide, respectively. These "extending oligonucleotides" may comprise binding sites for PCR primers, bar codes, type IIS restriction endonucleases, or any other nucleic acid sequence having a desired property. ("PCR," as used herein, refers to any method of duplex nucleic acid amplification regardless of the enzyme used.) As discussed in more detail below, extending oligonucleotides may also be labelled.

The extending oligonucleotide may be the product of a single oligonucleotide synthesis reaction. The extending oligonucleotide may be a mixture of the products of a plurality of oligonucleotide syntheses, the full-length products of which differ in sequence from each other. The products of five to 100 separate oligonucleotide synthesis may be pooled to form the extending oligonucleotide mixture.

Type IIS restriction enzymes have distinct DNA binding and cleavage domains; they recognize a specific sequence but cleave a defined distance away. *Reviewed in* Szybalski et al., Gene 100:13-26 (1991). Type IIS restriction enzymes useful for purposes of the present invention include, but are not limited to, *Aar*I, *Acc*36I, *Acl*WI, *Acu*I, *Alw*26I, *Alw*XI, *Alw*XI, *Asu*HPI, Bbsl , *Bbv*16I*, Bbv*I, *Bbv*II, *Bcc*I, *Bce*AI, *Bcef*I, *Bcg*I, *Bci*VI, *Bco*35I, *Bco*KI, *Bfil , Bful , Bfu*AI, *Bin*I, *Bmr*I, *Bpil, Bpm*I, *Bpu*AI, *Bpu*EI, *Bsa*I, *Bsc*AI, *Bse*3DI, *Bse*GI, *Bse*MI, *Bse*MII, *Bse*RI, *Bse*XI, *Bsg*I, *Bsm*AI, *Bsm*BI, *Bsm*FI, *Bso*31I, *Bso*MAI, *Bsp*6II, *Bsp*22I, *Bsp*28I, *Bsp*432I, *Bsp*CNI, *Bsp*MI, *Bsp*PI, *Bsp*VI, *Bsr*DI, *Bsr*EI, *Bst*6I, *Bst*71I, *Bst*F5I, *Bst*V1I, *BstV*2I, *Bthll, Bts*I, *Bsu*6I, *Cre*I, *Eam*1104I, *Ear*I, *Eci*I, *Eco*31I, *Eco*57I, *Eco*57MI, *Eco*112I, *Eco*125I, *Esp*3I, *Fau*I, Fokl, *Fsf*I, *Gsu*I, Hgal, *Hin*GUII, *Hph*I, *Hpy*II, *Ksp*632I, *Lla*I, *Lwe*I, *Mbo*II, *Mly*I, *Mme*I, *Mnl*I, *Ncu*I, *Ngo*BI, *Ngo*VIII, *Ple*I, *Ppsl, Ral*8I, *Rle*AI, *Sap*I, *Sce*I, *Sch*I, *Sfa*NI, Smul, *Sth*132I, Stsl, Taqll, *Tce*I, *Tsp*GWI, *Tth*111I, *Uba*1109I, and *Vpa*K32I.

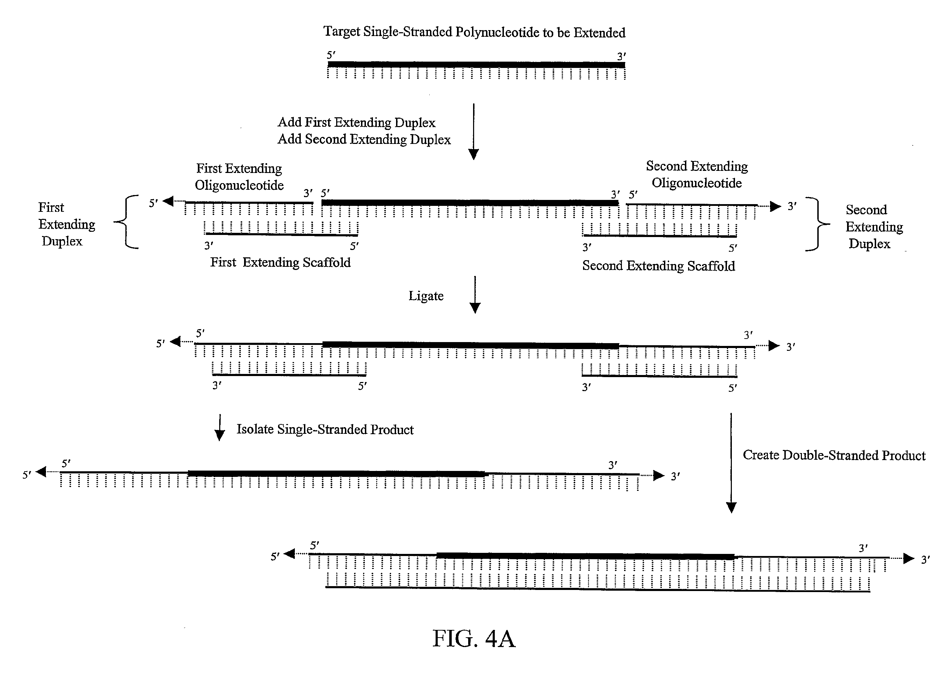

In FIG. 4A, extending oligonucleotides comprising binding sites for first and second PCR primers are ligated to the 3' and the 5' ends of a single stranded target polynucleotide, respectively. For simplicity, only one species of ss polynucleotide to be extended is shown in FIG. 4A, but the method may be used with mixtures of many different species of ss polynucleotides. In the method illustrated in FIG. 4A, the target ss polynucleotide ("target") is combined (e.g. mixed) with a pre-formed first extending duplex (comprising a first extending oligo annealed to a first extending scaffold oligo) and a pre-formed second extending duplex (comprising a second extending oligo annealed to a second extending scaffold oligo). In other methods, however, the individual oligonucleotides and polynucleotide may be added in any order, with or without pre-formation of any duplex species.

The extending scaffold oligos illustrated in FIG. 4A are complementary to the extending oligos along a portion of their (the extending scaffolds') length, and the complementary to the target polynucleotide along another portion of their (the extending scaffolds') length, such that when the target and the extending oligos are base paired to their respective extending scaffolds there are no "gaps" in the base pairing at the junctions of the extending oligos and the target. Duplex formation with the extending scaffolds brings the target polynucleotide and the extending oligonucleotides into proper orientation and proximity for efficient ligation. Such oligonucleotides are properly aligned for ligation. The extending oligos are then ligated to the target polynucleotide, for example by addition of T4 DNA ligase and appropriate co-factors, and incubation for 30 minutes at 37-42°C.

The scaffold oligonucleotide in FIG. 4A does not have bases beyond those complementary to either the extending oligos or the target. Such a scaffold oligonucleotide could be chemically synthesized. In other instances, however, additional bases that are not complementary to either the extending oligo or the target extend from the first and/or the second end of the scaffold oligonucleotide. In one instance, the scaffold oligonucleotide comprises a single-stranded fragment of genomic DNA.

After ligation, the single-stranded polynucleotide product may be isolated and used directly, or the complement may be synthesized to give a (partially) double-stranded product.

In one instance, in which the extending oligos comprise binding sites for type IIS restriction endonucleases, the double-stranded ligation product in FIG. 4A may be digested with type IIS restriction endonucleases to give a double-stranded DNA fragment suitable for cloning. The use of a different extending sequence at the 5' end from that used at the 3' end makes it possible to introduce different restriction sites at the two ends of the molecule, facilitating directional cloning.

In instances in which extending oligos comprise PCR binding sites, the double-stranded product illustrated in FIG. 4A may be amplified by PCR, or the isolated single-stranded product may amplified in a PCR reaction directly without the separate step of creating the duplex product.

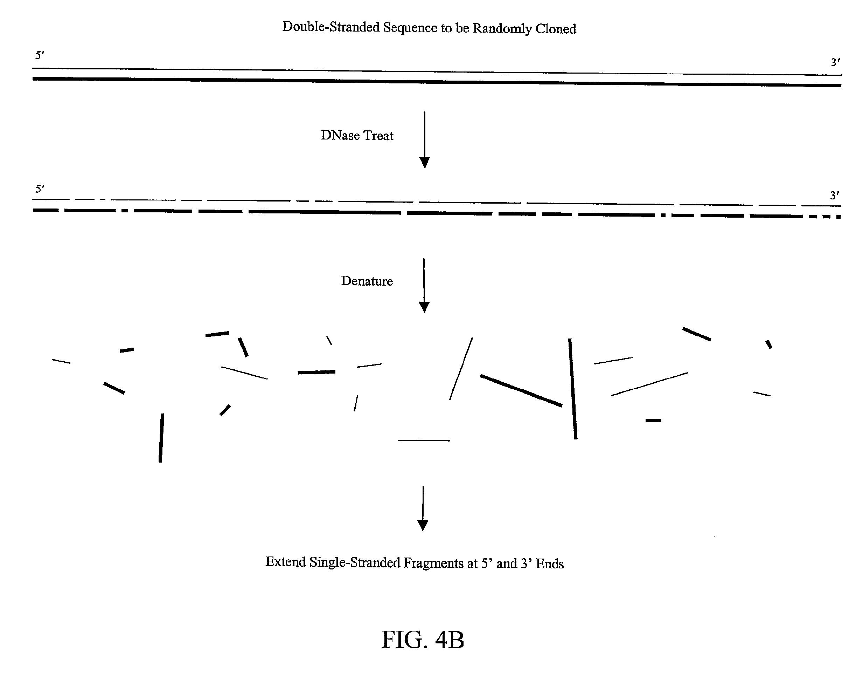

The use of a different extending sequence at the 5' end from that used at the 3' end in such embodiments makes it possible to have different PCR primer binding sites at the 5' and 3' ends. This reduces the chances of spurious intra-strand "hairpin" formation of the type that can result when the same primer binding sequence is present at both ends of DNA fragment to be amplified, in which the 3' end of each strand is necessarily the reverse complement of the 5' end. Single-stranded fragments can bend to allow the formation of a hairpin (antiparallel duplex between the 3' and 5' ends) that can interfere with subsequent manipulations, such as PCR amplification and restriction digestion. In addition, since hairpin formation depends on the length and sequence composition of the fragment, not all sequence fragments will be equally affected. This inequality can lead to skewing of results when a large mixture of fragments, differing significantly in the likelihood of hairpin formation, is used. Skewing is particularly harmful in procedures such as random cloning of genomic sequences (discussed with reference to FIG. 4B, *infra*) where it is desirable for the clone library to represent all the fragments of the genomic DNA being cloned equally. Fragments with different PCR primer binding sites at the ends are less subject to such hairpin-related artifacts.

In instances in which extending oligos comprise bar code sequences, the product polynucleotide comprising any given target single-stranded polynucleotide sequence may be detected by hybridization to an array containing an oligonucleotide complementary to the unique bar code sequence ligated to the target polynucleotide of interest.

FIG. 4B illustrates one practical application of the method illustrated in FIG. 4A, *i.e.,* random cloning of genomic DNA. In the application illustrated, a double-stranded DNA sample of interest is digested with DNase I for a time sufficient to give fragments of the desired size. The resulting randomly nicked double stranded DNA is then denatured to produce a mixture of single-stranded polynucleotides suitable for extension by the method illustrated in FIG. 4A.

A different kind of scaffold oligonucleotide is used for random cloning than would be used for extension of a single, known target sequence. When a single known sequence is to be extended according to the method of FIG. 4A, the short sticky ends of the scaffold oligonucleotides are synthesized to be complementary to the known sequence of the target polynucleotide. When randomly cloning genomic DNA, however, the sequence at the ends of the DNase fragments to be extended is variable and generally not known. The scaffold oligonucleotide is therefore synthesized with a mixture of all four bases at each position in the sticky end, to ensure that there is a complementary scaffold oligonucleotide for all possible sequences at the termini of the DNase fragment to be cloned. In one instance, the sticky end is seven nucleotides long, and thus each scaffold oligonucleotide mixture contains over 16,000 different sticky end sequences. In other instances, the randomized sticky end may be four, five, six, eight, ten, twelve or more nucleotides long.

The method of FIG. 4A is then followed using the mixture of scaffold oligonucleotides with randomized sticky ends, and using the mixture of genomic DNA fragments as the target polynucleotide. Extending sequences, optionally comprising type IIS restriction endonuclease binding sites, are ligated to the genomic DNA fragments. Complementary strands are synthesized, and the double-stranded product is digested with the appropriate restriction endonucleases. Such restriction fragments may be cloned into a vector in a defined orientation in embodiments in which the enzymes cleaving the two ends are different, providing termini after digestion that are different.

There is described herein a kinetic sampling ligation method that is used to label polynucleotides at either or both of the 5' or 3' ends.

There are also described herein kits to perform such labeling reactions.

When creating polynucleotides for use as genetic probes, it is often desirable to label the probe. However, existing methods used to label the 5' end differ significantly from the methods used to label the 3' end, owing to the inherent structural differences between the two ends. For example, a radioactive or fluorescent label can be enzymatically added at the 3' end of a polynucleotide using polymerase or terminal transferase reactions in the presence of labeled nucleotides. Labeling of the 5' end of a polynucleotide, in contrast, is frequently limited to radioactive labeling using a kinase reaction. Alternative chemical methods for labeling either the 5' or the 3' end of a polynucleotide exist, but such methods are often expensive and potentially damaging to the polynucleotide itself.

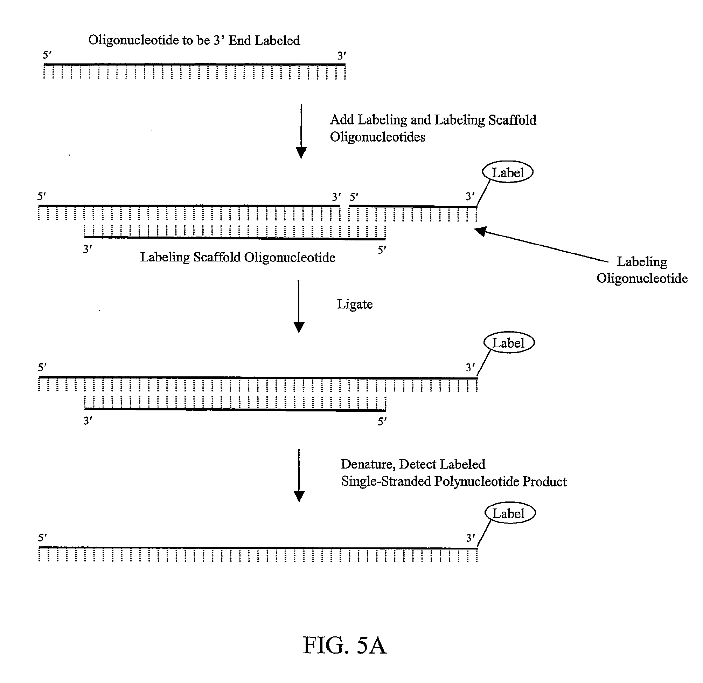

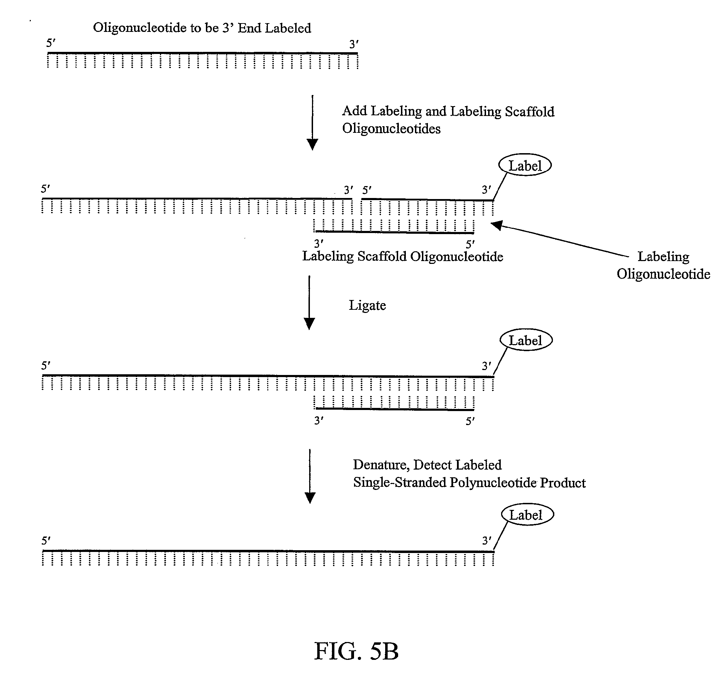

Methods for labeling either or both of the 5' or 3' ends of a polynucleotide are illustrated in FIGS. 5A and 5B and demonstrated in Examples 4 and 5. FIGS. 5A and 5B illustrate several methods for labeling the 3' end of a polynucleotide by sampling ligation. One of skill in the art would also be able to apply the same principle to adapt the method to label the 5' end of a polynucleotide. Multiplex labeling methods are not illustrated, but are within the scope of the present invention and involve labeling a plurality of different oligonucleotide species simultaneously in the same labeling reaction.

For the methods illustrated in FIGS. 5A and 5B, the labeling reaction is comprised of 1) an oligonucleotide or polynucleotide to be labeled at its 3' end, 2) a labeling oligonucleotide which is already labeled with the desired label, preferably at its 3' end, and 3) a labeling scaffold oligonucleotide. The labeling scaffold oligonucleotide plays the same role as the scaffold oligonucleotide in the method illustrated in FIG. 2A and described above, in that it provides complementary bases to properly align the oligonucleotide to be labeled and the labeling oligonucleotide for ligation.

In the method illustrated in FIG. 5A, the labeling scaffold has at its 3' end a region complementary to the oligonucleotide to be labeled long enough to form a stable duplex, and a region at its 5' end complementary to the labeling oligonucleotide that is not long enough to form a stable duplex under ligation conditions. In the method illustrated in FIG. 5B, the labeling scaffold has at its 5' end a region complementary to the labeling oligonucleotide long enough to form a stable duplex, and a region at its 3' end complementary to the oligonucleotide to be labeled that is not long enough to form a stable duplex under ligation conditions. In one embodiment, the labeling scaffold has a 7 nt region complementary to the labeling oligonucleotide and a 20 nt region complementary to the oligonucleotide to be labeled. In another embodiment, the labeling scaffold has a 7 nt region complementary to the oligonucleotide to be labeled and a 20 nt region complementary to the labeling oligonucleotide.

The labeling oligonucleotide can be made detectable by labeling it in any way that facilitates detection, such as by attachment of a detectable label.

Detectable labels include, e.g., a radionuclide, a fluorophore, a fluorescence resonance energy transfer ("FRET") tandem fluorophore, a FRET donor and/or acceptor, or a mass tag. Indirectly detectable labels include, e.g., an enzyme, a genotypic label, or a hapten.

The label may, for example, be a radionuclide, such as ³³P, ³²P, ³⁵S, and 3H.

The label may instead be a fluorophore. Commercially available fluorescent nucleotide analogues readily incorporated into the labeling oligonucleotides include, for example, Cy3-dCTP, Cy3-dUTP, Cy5-dCTP, Cy3-dUTP (Amersham Biosciences, Piscataway, New Jersey, USA), fluorescein-12-dUTP, tetramethylrhodamine-6-dUTP, Texas Red®-5-dUTP, Cascade Blue®-7-dUTP, BODIPY® FL-14-dUTP, BODIPY® ™R-14-dUTP, BODIPY® TR-14-dUTP, Rhodamine Green™-5-dUTP, Oregon Green® 488-5-dUTP, Texas Red®-12-dUTP, BODIPY® 630/650-14-dUTP, BODIPY® 6501665-14-dUTP, Alexa Fluor® 488-5-dUTP, Alexa Fluor@ 532-5-dUTP, Alexa Fluor@ 568-5-dUTP, Alexa Fluor® 594-5-dUTP, Alexa Fluor® 546-14-dUTP, fluorescein-12-UTP, tetramethylrhodamine-6-UTP, Texas Red®-5-UTP, Cascade Blue®-7-UTP, BODIPY® FL-14-UTP, BODIPY® TMR-14-UTP, BODIPY® TR-14-UTP, Rhodamine Green™-5-UTP, Alexa Fluor® 488-5-UTP, Alexa Fluor® 546-14-UTP (Molecular Probes, Inc. Eugene, OR, USA). Protocols are available for custom synthesis of nucleotides having other fluorophores. Henegariu et al., "Custom Fluorescent-Nucleotide Synthesis as an Alternative Method for Nucleic Acid Labeling," Nature Biotechnol. 18:345 - 348 (2000).

Other fluorophores available for post-synthetic attachment include, *inter alia,* Alexa Fluor® 350, Alexa Fluor® 532, Alexa Fluor® 568, Alexa Fluor® 594, Alexa Fluor® 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA), and Cy2, Cy3.5, Cy5.5, and Cy7 (Amersham Biosciences, Piscataway, NJ USA, and others).

FRET tandem fluorophores may also be used, such as PerCP-Cy5.5, PE-Cy5, PE-Cy5.5, PE-Cy7, PE-Texas Red, and APC-Cy7.

Labels that are detectable by mass spectrometry, "mass tags," may also be used. Mass tags can be designed to provide hundreds of mass spectrally distinguishable species, allowing highly multiplexed reactions, and can be designed to be cleavable, typically photochemically cleavable, from the labeled nucleic acid, simplifying analysis. See, e.g., Kokoris et al., Mol Diagn. 5(4):329-40 (2000) and Pusch et al., Pharmacogenomics 3(4):537-48 (2002).

The labeling oligonucleotide may instead or in addition include at least one indirectly detectable label.

For example, the oligonucleotide may include an enzyme.

Enzymes useful for colorimetric detection are well known, and include alkaline phosphatase, β-galactosidase, glucose oxidase, horseradish peroxidase (HRP), and urease. Typical substrates for production and deposition of visually detectable products include o-nitrophenyl-beta-D-galactopyranoside (ONPG); o-phenylenediamine dihydrochloride (OPD); p-nitrophenyl phosphate (PNPP); p-nitrophenyl-beta-D-galactopyranoside (PNPG); 3',3'-diaminobenzidine (DAB); 3-amino-9-ethylcarbazole (AEC); 4-chloro-1-naphthol (CN); 5-bromo-4-chloro-3-indolyl-phosphate (BCIP); ABTS®; BluoGal; iodonitrotetrazolium (INT); nitroblue tetrazolium chloride (NBT); phenazine methosulfate (PMS); phenolphthalein monophosphate (PMP); tetramethyl benzidine (TMB); tetranitroblue tetrazolium (TNBT); X-Gal; X-Gluc; and X-Glucoside.

Enzymes may also be used for luminescent detection.

For example, in the presence of hydrogen peroxide (H₂O₂), horseradish peroxidase (HRP) can catalyze the oxidation of cyclic diacylhydrazides, such as luminol, with subsequent light emission. Strong enhancement of the light emission can be produced by enhancers, such as phenolic compounds. Thorpe et al., Methods Enzymol. 133:331-53 (1986); Kricka et al., J. Immunoassay 17(1):67-83 (1996); and Lundqvist et al., J. Biolumin. Chemilumin. 10(6);353-9 (1995). Kits for such chemiluminescent and enhanced chemiluminescent detection of nucleic acids are available commercially.

Labels can be attached to the end of the labeling oligonucleotide or internally, so long as the labels do not interfere with annealing of the labeling oligonucleotide to the labeling scaffold or the ligation of the labeling oligonucleotide to the oligonucleotide to be labeled. In one embodiment, ligation reactions are performed under conditions that permit stable duplex formation between the oligonucleotide to be labeled and the labeling scaffold, but not between the labeling oligonucleotide and the labeling scaffold, as discussed above in relation to FIG. 5A. In one embodiment, ligation is performed using T4 DNA ligase at 37°C - 42°C for 30 minutes. The amount of product formed by ligation of the labeling oligonucleotide to the oligonucleotide to be labeled is then determined, for example by PAGE.

In one embodiment, labeling reactions according to the present invention are performed in multiplex, *i.e*. with a plurality of oligonucleotide species to be labeled in a single ligation mixture. The plurality of oligonucleotide species comprises two or more oligonucleotide species, such as 24, 96 or more.

The efficiency of labeling of oligonucleotides may optionally be assessed by hybridization to an array of complementary oligonucleotides or by any other suitable technique.

Labeled oligonucleotides may optionally be purified by preparative denaturing PAGE or denaturing agarose gel electrophoresis. Preparative electrophoresis may be performed on a single labeled oligonucleotide, a plurality of oligonucleotides labeled in a multiplex reaction, or a mixture of the products of two or more labeling reactions.

The following examples are provided by way of illustration, and not limitation.

### EXAMPLE 1

### Construction of Polynucleotides Suitable for Use as Genetic Probes Using Unpurified Products of a Single Oligonucleotide Synthesis Reaction

A fluorescently labeled, gel purified 37 nt central oligonucleotide is annealed to a gel purified 51 nt scaffold oligonucleotide to form a duplex over the entire length of the central oligonucleotide, with 7 nt remaining single-stranded at each end of the scaffold. These single-stranded 7 nt regions are referred to as sticky ends.

A series of 48 pairs of first and second oligonucleotides are synthesized and used without purification. The 48 pairs of oligonucleotides vary in sequence, length, and purity. The length of the first oligonucleotide varies from 25 nt to 32 nt, and length of second from 51 nt to 58 nt. Based on an estimated 98% efficiency of single base addition during oligonucleotide synthesis, the yield of full-length product should be at most 52% for 32-mers and 30% for 58-mers, with the remainder being various truncated forms. However, in actual syntheses the yield of full-length product oligonucleotides can be significantly lower, and varies dramatically. Unless otherwise indicated, in this and other examples, all oligonucleotides are synthesized so that a phosphate group is present at the 5' terminus.

Each full-length first oligo has at its 3' end a region of 7 nt complementary to the 7 nt sticky end at the 3' end of the scaffold oligonucleotide, and each full-length second oligo has at its 5' end a region of 7 nt complementary to the 7 nt sticky end at the 5' end of the scaffold oligonucleotide.

Separate ligation reactions are performed to ligate each of the 48 pairs of first and second oligos to the central oligonucleotide of the core duplex. First and second oligos are added simultaneously (1 µM each) to a solution of pre-formed core duplex between the central and scaffold oligonucleotides (0.1 µM each) in ligase buffer consisting of 50 mM Tris acetate (pH 7.6), 10 mM Mg acetate, 5 mM DTT, 25 µg/ml bovine serum albumin (BSA), and 1 mM ATP. All concentrations listed in this and other examples are concentrations in the final reaction mixture after all components have been added. The ligation reaction mixture is heated to 42°C, T4 DNA ligase (New England Biolabs, Inc., Beverly, Mass., USA) is added to 8000 units/ml, and the reaction is allowed to proceed for 30 minutes. As used herein, a unit of ligase is a cohesive end ligation unit as defined by New England Biolabs. Reaction products are then resolved by denaturing 7M urea polyacrylamide gel electrophoresis. Bands are detected by scanning the gel with a fluorescent scanner.

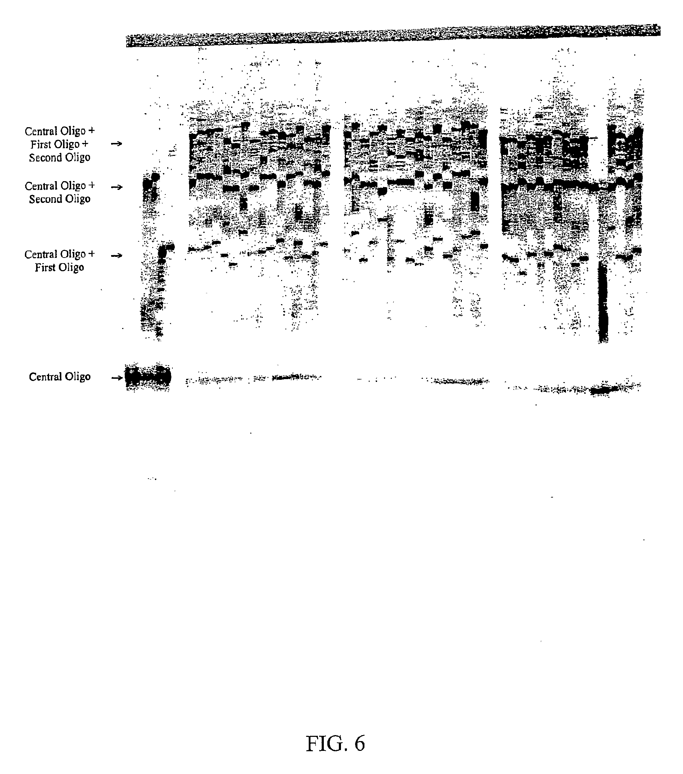

Results are shown in FIG. 6. Bands represent either unligated central oligonucleotide or central oligonucleotide ligated to first, second or both oligos, as indicated. The leftmost five lanes are control lanes loaded (from left to right) with unligated central oligonucleotide, central oligonucleotide ligated to one species of second oligo, central oligonucleotide ligated to a different species of second oligo, central oligonucleotide ligated to one species of first oligo, and central oligonucleotide ligated to a different species of first oligo. The lane to the right of the controls is empty, and is followed by 48 lanes loaded with products of ligation reactions performed with core duplex and a series of pairs of first and second oligos, different for each lane. Lanes to the right of those loaded with the 16th and 32nd ligation reaction mixtures are left unloaded.

Forty six of the 48 pairs of first and second oligos are successfully ligated into full-length polynucleotide products, with little variation in the proportion of full-length product formed despite differences in the sequence, length and purity among the first and second oligonucleotide synthesis reaction product mixtures used.

### EXAMPLE 2

### Purification of the Full-Length Ligation Product

Full-length product polynucleotides constructed according to the method of the present invention may optionally be purified away from other components of the ligation reaction, including excess of first and second oligonucleotides and ligation byproducts. Polynucleotides may be purified by denaturing polyacrylamide gel electrophoresis. The presence of urea in the denaturing gel interferes with detection of DNA using standard DNA staining agent such as ethidium bromide or SYBR® Green (Molecular Probes, Inc., Eugene, Oregon, USA) unless the urea is removed by soaking the gel in water or buffer. The method presented involves direct visualization of the products of the reaction, allowing bands to be cut from the gel without the need to soak the gel to remove urea.

In one experiment, 10 M of first and second oligonucleotides are mixed with 2 M of core duplex complex in a 50 L reaction volume. The reaction proceeds as described in EXAMPLE 1. An equal volume of formamide is added at the end of the reaction. This mixture is preheated for 2 min at 95°C and then cooled on ice, and loaded onto a 10% preparative denaturing polyacrylamide gel. After separation is complete the gel is placed between layers of plastic cling wrap, placed over a fluorescent TLC plate and illuminated with 254 nm UV light from a handheld UV lamp. Polynucleotides are visible due to shadowing of UV light, *i.e*. decreased fluorescence of the TLC plate beneath areas of the gel where the polynucleotides are located. Bands with mobilities corresponding to the full-length polynucleotide are visible in all ligation reactions described in EXAMPLE 1.

If the central oligonucleotide is labeled with fluorescein it is possible, by placing the gel on a UV light source and detecting fluorescence, to detect lesser amounts of full-length polynucleotide than is possible with the method described above. Other effective combinations of illumination and filters can optionally be used to visualize the fluorescein label. Whatever the means of detection, bands corresponding to full-length polynucleotide products are cut from gel and extracted.

### EXAMPLE 3

### Inhibition of Ligation by Nonligatable Competing Oligonucleotides Under Stable Ligation Conditions and Unstable Ligation Conditions

A core duplex with 7 nt sticky ends is created as in Example 1. A different core duplex with two 18 nt sticky ends is created by annealing the same central oligonucleotide with a 73 nt scaffold oligonucleotide. The sticky ends of both scaffold oligonucleotides are fully complementary to the 3' end of the first oligo and the 5' end of the second oligo. The core duplexes are formed by adding a two-fold molar excess of scaffold to the fluorescently labeled central oligonucleotide and heating to 80°C for 2 min., followed by 46°C for an additional 30 minutes. Core duplexes are used at approximately 0.1 µM in the final ligation reactions.

First oligo, second oligo, both oligos or neither oligo are added to the core duplex with 7 nt sticky ends and, in separate reactions, to the core duplex with 18 nt sticky ends, in each case to a concentration of 1 µM (a ten-fold molar excess over the core duplexes). The result is eight separate ligation reactions comprising one of the two core duplexes with either the first, the second, both or neither oligo(s).

An additional ligation reaction is performed, with conditions identical to each of those described above except that nonligatable competing oligonucleotides are added at a ten-fold molar excess (10 µM) over the first and second oligos. These nonligatable oligos simulate contaminants. For the first oligo, the nonligatable competing oligo is identical to the full-length first oligo except that it lacks one nucleotide at the 3' end. This competing oligo is referred to as the "first (n-1) oligo." For the second oligo, the nonligatable competing oligo is identical to the full-length second oligo except that it lacks a phosphate group at the 5' end. This competing oligo is referred to as the "second (5'-OH) oligo." Reactions involving only the first oligo are challenged only with the first (n-1) oligo, and reactions involving only the second oligo are challenged only with the second (5'-OH) oligo. Reactions involving both the first and second oligos are challenged with both nonligatable competing oligos. Control ligations containing only nonligatable competing oligos are also performed, *i.e*. ligations like those mentioned previously except that no first or second oligo is added.

Four hundred units of T4 DNA ligase are added to each 50 µL reaction. ATP and BSA are added to 1 mM and 25 µg/ml final concentrations, respectively. Duplicates of all of the aforementioned ligation reactions are performed, one at 37°C and the other at 42°C. Time points are withdrawn at 10 and 30 minutes and stopped by addition of three volumes of formamide containing bromphenol blue. Samples are then loaded onto a 10% PAGE gel containing 7M urea. Gel results are quantified using a Molecular Dynamics (Amersham Biosciences) fluorescent scanner. After the scan, the intensity of each band is determined by integrating the area under the corresponding peak. The average of the 10 and 30 minute results is presented in TABLE 1.

**TABLE 1**

| Percent Formation of Full-Length Product Polynucleotide | | | |
|---|---|---|---|
| Sticky End (Temperature) | Competing First Oligonucleotide | Competing Second Oligonucleotide | Both Competing Oligonucleotides |
| 7 nt (37°C) | 100% | 83% | 77% |
| 7 nt (42°C) | 100% | 88% | 91% |
| 18 nt (37°C) | 22% | 8% | (<2.5%) |
| 18 nt (42°C) | 32% | 7% | 3% |

Table 1 presents the amount of full-length ligation product obtained in reactions containing a ten-fold molar excess of nonligatable competing oligonucleotides as a percentage of the amount of full-length ligation product obtained in identical reactions lacking the competing oligonucleotides. At both 37°C and 42°C, the reactions involving unstable duplex formation between the first (and second) oligos and the scaffold, *i.e*. those involving 7 nt sticky ends, are significantly less inhibited by the presence of simulated contaminants. A ten-fold excess of the first (n-1) oligo has no effect on ligation of the first oligo to the central oligonucleotide, and a similar excess of the second (5'-OH) oligo decreases ligation of the second oligo to the central oligonucleotide only 12-17%. The presence of both simulated contaminants reduces full-length product formation 19-23% for reactions involving unstable 7 nt sticky ends.

In contrast, ligations involving 18 nt sticky ends are severely impaired by simulated contaminants. A ten-fold excess of the first (n-1) oligo reduces ligation of the first oligo to the central oligonucleotide by 68-78%, more than three-fold. A similar excess of the second (5'-OH) oligo decreases ligation of the second oligo to the central oligonucleotide from 12 to 14-fold. The presence of both simulated contaminants reduces full-length product formation by 30-fold for reactions at 42°C, and to below the limits of detection (>40-fold) for reactions at 37°C.

An additional and unexpected benefit of the use of unstable ligation is that very little adenylation of the central oligonucleotide occurs, whereas significant adenylation occurs under stable ligation conditions. Adenylation is an undesired side reaction that involves transfer of an AMP moiety from a ligase-AMP intermediate to the 5' end of one of the oligonucleotides to be ligated. In a successful ligation reaction, the AMP moiety is released upon formation of the phosphodiester bond between the two oligonucleotides, *i.e*. the ligation of the strands. However, under some conditions the ligation reaction fails to proceed to the formation of a phosphodiester bond, leaving an adenylated 5' end. Such adenylated strands can be identified on the basis of their distinctive mobility in PAGE.

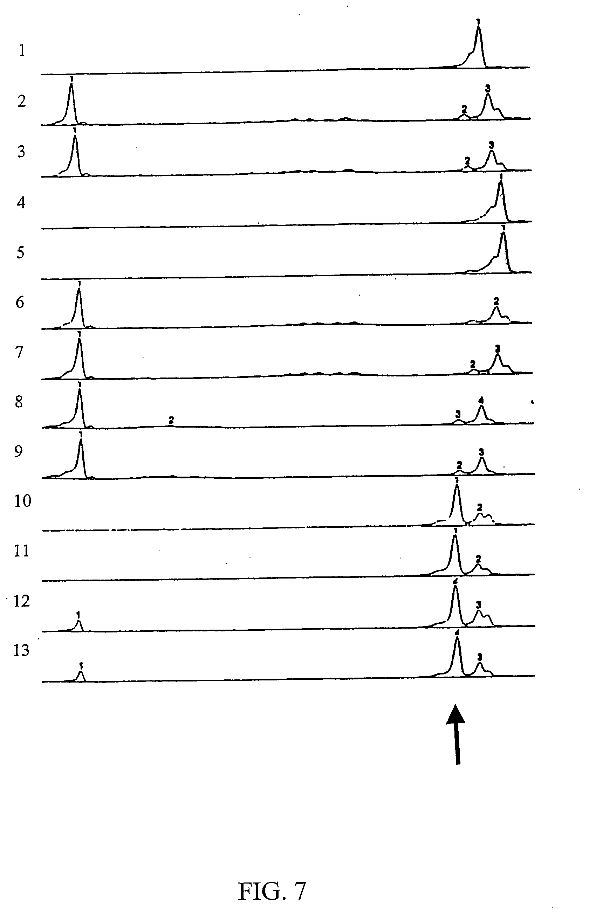

FIG. 7 shows densitometric scans of lanes from a gel loaded with ligation reaction products, illustrating the formation of adenylated central oligonucleotide (indicated by the arrow). The numbers refer to lanes on the gel. The leftmost peak in lanes 2, 3, 6-9, 12 and 13 is the full-length polynucleotide product, and the rightmost peak in all lanes is the central oligonucleotide. Lanes 1-7 show the products of ligation reactions performed using a core duplex with a 7 nt sticky end, which forms unstable duplexes with the first and second oligos under reaction conditions (37°C). No appreciable quantity of adenylated central oligonucleotide is formed. Lanes 8-13 show the products of ligation reactions performed using a core duplex with an 18 nt sticky end, which forms stable duplex with the first and second oligos under reaction conditions. Only reactions involving stable duplex formation and including the truncated form of the first oligo, shown in lanes 10-13, show significant formation of the adenylated central oligonucleotide. Up to 80% of the central oligonucleotide is adenylated in reactions shown in lanes 10-13. Once adenylated, such oligonucleotides cannot subsequently be ligated at their 5' end.

Without intending to be bound by theory, it is possible that the first (n-1) oligo in the 18 nt sticky end reaction, which can form a 17 bp duplex with the scaffold oligonucleotide, forms a stable complex, albeit with a single base gap between the ends that would otherwise be ligated. This gapped duplex may permit T4 DNA ligase binding and AMP transfer to the 5' end of the central oligonucleotide (adenylation), but not phosphodiester bond formation. The analogous complex between the first (n-1) oligonucleotide in the 7 nt sticky end reaction would be able to form only a 6 bp duplex, which may be too unstable to permit binding and AMP transfer by T4 DNA ligase. Whatever the molecular mechanism, the method of the present invention has the advantage that the central oligonucleotide is not irreversibly inactivated by adenylation when unstable ligation conditions (7 nt sticky ends) are used, as it is when stable ligation conditions (18 nt sticky ends) are used.

### EXAMPLE 4

### Labeling of the 3' or 5' Ends of Unpurified Oligonucleotides by Kinetic Sampling Ligation

Six different unpurified oligonucleotides are labeled at their 3' ends by kinetic sampling ligation and six others are labeled at their 5' ends. FIGS. 5A and 5B show two methods of labeling the 3' end of an oligonucleotide. No schematic is presented for the analogous method for labeling the 5' end of an oligonucleotide.

To label the 3' ends of the first six oligonucleotides, six separate 27 nt labeling scaffold oligonucleotides and one labeling oligonucleotide are synthesized. Each of the scaffold oligonucleotides contains a different 20 nt sequence at its 3' end complementary to the 3' end of one of the six oligonucleotides to be labeled, and a 7 nt common sequence at its 5' end complementary to the 5' end of the labeling oligonucleotide. The labeling oligonucleotide for 3' end labeling is 20 nt long and has fluorescein attached at its 3' end.

To label the 5' end of the second six oligonucleotides (no schematic shown), six separate 27 nt labeling scaffold oligonucleotides and one labeling oligonucleotide are synthesized. Each of the scaffold oligonucleotides contains a different 20 nt sequence at its 5' end complementary to the 5' end of one of the six oligonucleotides to be labeled, and a 7 nt common sequence at its 3' end complementary to the 3' end of the labeling oligonucleotide. The labeling oligonucleotide for 5' end labeling is 10 nt long and has fluorescein attached at its 5' end.

Phosphate groups are chemically coupled to the 5' ends of all oligonucleotides used in this example during synthesis, with the exception of those oligonucleotides that are labeled at their 5' end with fluorescein. No oligonucleotides used in this example are purified with the exception of the labeling oligonucleotides.

In separate reactions, each of the six oligonucleotides to be labeled at their 3' ends, ranging in length from 52 nt to 58 nt, and each of the six oligonucleotides to be labeled at their 5' ends, ranging in length from 25 nt to 32 nt, is annealed to the corresponding labeling scaffold oligonucleotide. Oligonucleotides to be labeled are used at 0.1 M and labeling scaffold oligonucleotides are used at 0.5 M. Ligase buffer is added, and the 3' end or 5' end labeling oligos are added to their respective reactions to 0.2 M. Reactions are heated to 42°C and then 400 units of T4 DNA ligase is added to each 50 µL reaction. After incubation for 30 min. at 42°C, reaction products are analyzed by 10% PAGE.

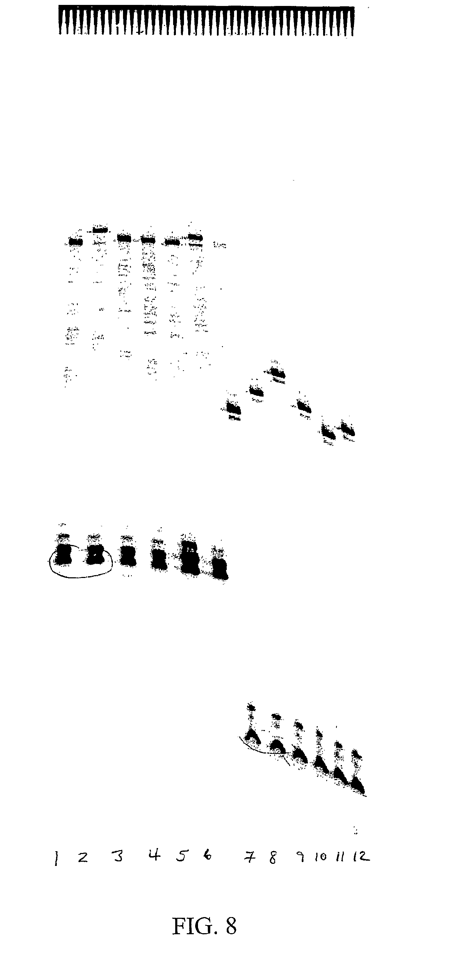

Results of the experiment are presented in FIG. 8. Lanes 1-6 are the products of the six 3' end labeling reactions, and lanes 7-12 are the products of the six 5' end labeling reactions. The lower clusters of bands in each lane are unreacted labeling oligonucleotide, and the dark upper bands in each lane are the desired labeled ligation products. All 12 oligonucleotides are labeled equally well regardless of which end is being labeled. Since only oligonucleotides with perfect ends can be ligated, labeling by sampling ligation as described produces only one specific ligation product for each labeling reaction. Gaps will remain between any contaminating truncated oligonucleotides and the labeling oligonucleotide when annealed to the labeling scaffold oligonucleotide, preventing ligation, and thus labeling, of the truncates.

### EXAMPLE 5

### Multiplex Labeling of the 3' or 5' Ends of Unpurified Oligonucleotides by Kinetic Sampling Ligation

In the multiplex aspect of the labeling reaction of the present invention, oligonucleotides are pooled and labeling is performed on the mixture (containing a plurality of oligonucleotides) in a single reaction, rather than on each oligonucleotide in a separate reaction.

Over five hundred different oligonucleotides are labeled at their 3' ends in a series of 24-plex kinetic sampling ligation reactions. The procedure for multiplex kinetic sampling ligation labeling the 5' end is not described but is performed by analogy with Example 4

(above). Design, annealing and ligation of oligonucleotides is performed as in Example 4, and as illustrated in FIG. 5B, with the following modifications.

The labeling oligonucleotide is 21 nt long, with a fluorescent dye label at its 3' end. The scaffold oligonucleotide is 28 nt long, and its 5' end is complementary to the entire length of the labeling oligonucleotide. When annealed with the labeling oligonucleotide there remains a 7 nt single stranded region at the 3' end of the scaffold oligonucleotide.

The oligonucleotides to be labeled are 28 bases long, with the 3'-most 7 nt of all oligonucleotides being identical. This 7 nt region is complementary to the 3'-most 7 nt of the scaffold oligonucleotide (here also termed a "labeling scaffold'). The result is that all oligonucleotides to be labeled differ in the 21 nt at their 5' end, but all can anneal to the same scaffold oligonucleotide at their 3' ends.

The labeling oligonucleotide is annealed to the scaffold oligonucleotide in a 100 µl reaction containing 27 µM labeling oligonucleotide, 32 µM labeling scaffold oligonucleotide and 4.1X T4 probe buffer. 10X T4 probe buffer comprises 500 mM Tris Acetate pH 7.6, 100 mM Mg Acetate and 50 mM DTT. The annealing reaction is heated to 80°C for two minutes, then 37°C for 30 minutes and finally chilled on ice. The duplex resulting from the pairing of the labeling oligonucleotide and the scaffold oligonucleotide is referred to as the labeling core duplex.

The reaction is diluted to 10 µM labeling core duplex in 1.5X T4 probe buffer. A reaction is then prepared containing 4 µM labeling core duplex, 2 µM total oligonucleotide to be labeled and 1X T4 probe buffer. For a 24-plex labeling reaction, each individual oligonucleotide to be labeled is present at approximately 83 nM. The reaction mixture is heated to 42°C, T4 DNA ligase is added to 4 units/µl, and the reaction is incubated at 42°C for 30 minutes. The ligation reaction is then heated to 70°C for 10 minutes and cooled to 4°C. The ligation produces a set of 49 nt oligonucleotides with fluorescent labels at their 3' ends and a 21 nt variable region at their 5' ends. The products of all of the 24-plex kinetic sampling labeling reactions are pooled and purified by PAGE.

The relative efficiency of labeling of the separate oligonucleotide sequences is determined by hybridizing the mixture to an array of oligonucleotides. The array comprises a series of oligonucleotides immobilized at separate locations on a solid support. The sequences of the oligonucleotides bound to the array are chosen to be complementary to those in the mixture. The intensity of fluorescence signal at each location reflects the efficiency of labeling of the oligonucleotide complementary to the oligonucleotide immobilized at that particular location.

The multiplex-labeled oligonucleotide sample is allowed to hybridize to the oligonucleotide array overnight in 6X SSPE (60 mM sodium phosphate, pH 7.4, 6 mM disodium EDTA, 900 mM sodium chloride) at 40°C. The arrays are then washed and scanned to quantify fluorescence signal intensity at each location.

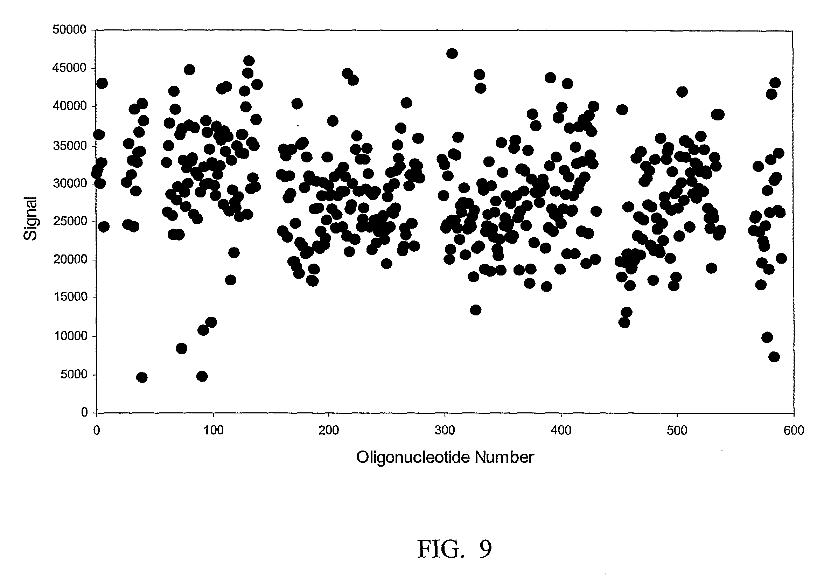

FIG. 9 shows the results of the array hybridization, with fluorescence intensity, in arbitrary units, plotted for each sequence. Each sequence is assigned an arbitrary numerical designation for ease of presentation of the data. Not all numbers are used in the numerical designations, *i.e*. there are gaps in sequence numbering. FIG. 9 shows that signal intensity is relatively uniform for all sequences, indicating relatively uniform labeling efficiency for the sequences examined.

As a further test of the efficacy of 24-plex kinetic sampling ligation labeling, one group of oligonucleotides is labeled using both the single-plex and 24-plex methods. The products of the single-plex reactions are pooled and hybridized to any array of complementary oligonucleotides, as described above, as are the products of the 24-plex labeling,

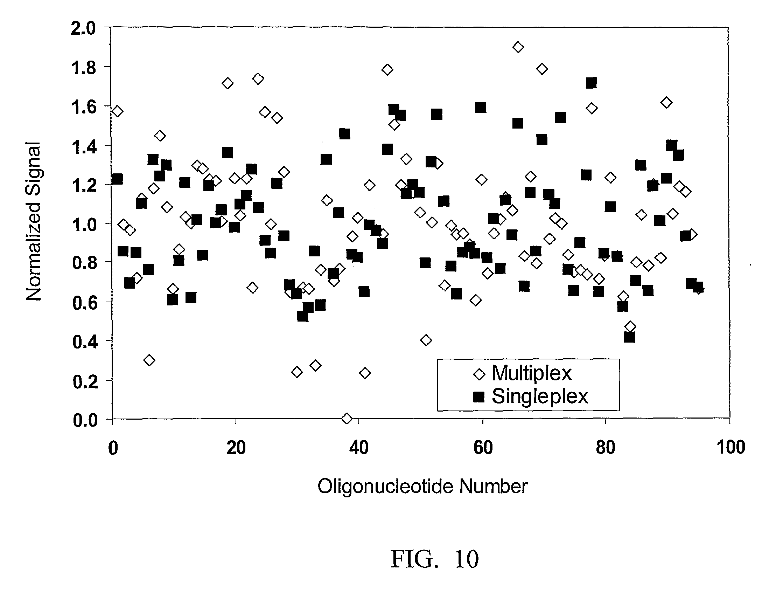

The signal intensity distribution for both sets of labeled oligonucleotides is shown at FIG. 10. The normalized signal is plotted for each oligonucleotide: normalized values are calculated by dividing the fluorescence signal intensity (in arbitrary units) for each oligonucleotide by the mean value for all oligonucleotides labeled the same way (*i.e*. singleplex or multiplex). The distribution of signal intensities is comparable between the individually labeled oligonucleotides and those labeled in 24-plex, demonstrating that oligonucleotides can be satisfactorily labeled in 24-plex reactions with consequent savings in effort and expense. It is likely that more than 24 oligonucleotides may also be labeled in multiplex.

Unless specifically stated, no step of the method of this invention requires any particular order of addition of materials, or order of performance of steps.

As used herein, reference to the presence of a specific number of oligonucleotides or sequences (*e.g.* one, two, etc.) refers to the number of different species present, rather than the number of molecules present.

Examples are intended to illustrate the invention and do not by their details limit the scope of the claims of the invention. While preferred illustrative embodiments of the present invention are described, it will be apparent to one skilled in the art that various changes and modifications may be made therein without departing from the invention as defined by the claims.

## Claims

1. A method of constructing at least one species of product polynucleotide using populations of truncate-containing oligonucleotide synthesis products, the method comprising:
ligating together a central oligonucleotide, at least one species of first oligonucleotide, and at least one species of second oligonucleotide,
the first, central and second oligonucleotides being annealed to a common scaffold oligonucleotide during ligation,
the scaffold oligonucleotide being complementary to the entire length of the central oligonucleotide, such that the duplex formed is stable under the ligation conditions, and the scaffold oligonucleotide being complementary to the first and second oligonucleotides only over a limited number of nucleotides, such that the duplexes formed are not stable under the ligation conditions, and wherein said scaffold oligonucleotide is subtemplate in length.

2. The method of claim 1, the method further comprising:
partially duplexing a scaffold oligonucleotide of subtemplate length having a first terminus and a second terminus with a central oligonucleotide that has a 5' terminus and a 3' terminus, such that a single-stranded region is left at both the first and second termini of the scaffold oligonucleotide; and
ligating a first and a second oligonucleotide, respectively, to the 5' and 3' termini of said central oligonucleotide by sampling respective first and second populations of truncate-containing oligonucleotide synthesis products with the single-stranded regions at the termini of said partially duplexed scaffold oligonucleotide wherein the first oligonucleotide includes a region perfectly complementary in sequence to the single-stranded region at the first terminus of said scaffold oligonucleotide and the second oligonucleotide includes a region perfectly complementary in sequence to the single-stranded region at the second terminus of said scaffold oligonucleotide.

3. The method of claim 2, wherein said partial duplexing and said ligating are performed in a single step.

4. The method of claim 2 or claim 3, wherein each of the single-stranded regions at the termini of the scaffold oligonucleotide is no more than 10 nucleotides long.

5. The method of claim 4, wherein each of the single-stranded regions at the termini of the scaffold oligonucleotide is no more than 7 nucleotides long.

6. The method of claim 5, wherein each of the single-stranded regions at the termini of the scaffold oligonucleotide is no more than 5 nucleotides long.

7. The method of any one of claims 2 to 6, wherein said first sampled population includes the products of a first plurality of oligonucleotide syntheses, the full-length synthesis products of at least two of said first plurality of syntheses being different in sequence, and said second population includes the products of a second plurality of oligonucleotide syntheses, the full-length synthesis products of at least two of said second plurality of syntheses being different in sequence.

8. The method of any one of claims 1 to 7, further comprising a step of size separating the product polynucleotide from the scaffold oligonucleotide.

9. The method of any one of claims 1 to 8, wherein the conditions of the ligating step include a temperature of at least 30°C.

10. The method of claim 9, wherein the conditions of the ligating step include a temperature of at least 42°C.

11. The method of claim 10, wherein the conditions of the ligating step include a temperature of at least 50°C.

12. The method of any one of claims 1 to 11, wherein the product polynucleotide extends at least 10 nucleotides beyond each of the termini of the scaffold oligonucleotide.

13. The method of claim 12, wherein the product polynucleotide extends at least 25 nucleotides beyond each of the termini of the scaffold oligonucleotide.

14. The method of claim 13, wherein the product polynucleotide extends at least 75 nucleotides beyond each of the termini of the scaffold oligonucleotide.

15. The method of any one of claims 1 to 14, wherein the scaffold oligonucleotide is partially duplexed to the central oligonucleotide, such that a single-stranded region is left at both the first and second termini of the scaffold oligonucleotide, prior to contacting the scaffold and central oligonucleotides with the first and second oligonucleotides.

16. The method of any one of claims 1 to 15, wherein the product polynucleotide is labeled.

## Patentansprüche

1. Verfahren zum Konstruieren mindestens einer Spezies eines Produktpolynukleotids unter Verwendung von Populationen von Trunkate enthaltenden Oligonukleotid-Syntheseprodukten, wobei das Verfahren Folgendes umfasst:
Zusammenligieren eines zentralen Oligonukleotids, mindestens einer Spezies eines ersten Oligonukleotids und mindestens einer Spezies eines zweiten Oligonukleotids,
wobei das erste, das zentrale und das zweite Oligonukleotid während der Ligation zu einem gemeinsamen Gerüst-Oligonukleotid fusioniert werden,
wobei das Gerüst-Oligonukleotid zu der gesamten Länge des zentralen Oligonukleotids komplementär ist, so dass der gebildete Doppelstrang unter den Ligationsbedingungen stabil ist, und das Gerüst-Oligonukleotid zu dem ersten und dem zweiten Oligonukleotid nur über eine begrenzte Anzahl von Nukleotiden komplementär ist, so dass die gebildeten Doppelstränge unter den Ligationsbedingungen nicht stabil sind, und wobei die Länge des Gerüst-Oligonukleotids unter der der Matrize liegt.

2. Verfahren nach Anspruch 1, wobei das Verfahren ferner Folgendes umfasst:
teilweise Duplizierung eines Gerüst-Oligonukleotids einer Matrizenlänge mit einem ersten Ende und einem zweiten Ende mit einem zentralen Oligonukleotid mit einem 5'-Ende und einem 3'-Ende, so dass eine einsträngige Region sowohl an dem ersten als auch an dem zweiten Ende des Gerüst-Oligonukleotids verbleibt; und
Ligieren eines ersten und eines zweiten Oligonukleotids entsprechend an die 5'-und 3'-Enden des zentralen Oligonukleotids durch Abtasten entsprechender erster und zweiter Populationen von Trunkate enthaltenden Oligonukleotid-Syntheseprodukten mit den einsträngigen Regionen an den Enden des teilweise duplexgebildeten Gerüst-Oligonukleotids, wobei das erste Oligonukleotid eine Region aufweist, die in ihrer Sequenz absolut komplementär ist zu der einsträngigen Region an dem ersten Ende des Gerüst-Oligonukleotids, und wobei das zweite Oligonukleotid eine Region aufweist, die in ihrer Sequenz absolut komplementär ist zu der einsträngigen Region an dem zweiten Ende des Gerüst-Oligonukleotids.

3. Verfahren nach Anspruch 2, wobei die teilweise Duplizierung und das Ligieren in einem einzigen Schritt ausgeführt werden.

4. Verfahren nach Anspruch 2 oder 3, wobei jede der einsträngigen Regionen an den Enden des Gerüst-Oligonukleotids nicht länger ist als zehn Nukleotide.

5. Verfahren nach Anspruch 4, wobei jede der einsträngigen Regionen an den Enden des Gerüst-Oligonukleotids nicht länger ist sieben Nukleotide.

6. Verfahren nach Anspruch 5, wobei jede der einsträngigen Regionen an den Enden des Gerüst-Oligonukleotids nicht länger ist fünf Nukleotide.

7. Verfahren nach einem der Ansprüche 2 bis 6, wobei die erste abgetastete Population die Produkte einer ersten Mehrzahl von Oligonukleotid-Synthesen aufweist, wobei Syntheseprodukte der ganzen Länge mindestens zwei der ersten Mehrzahl von Synthesen sich in der Sequenz unterscheiden, und wobei die zweite Population die Produkte einer zweiten Mehrzahl von Oligonukleotid-Synthesen aufweist, wobei Syntheseprodukte der ganzen Länge mindestens zwei der zweiten Mehrzahl von Synthesen sich in der Sequenz unterscheiden,

8. Verfahren nach einem der Ansprüche 1 bis 7, ferner umfassend einen Schritt der Größentrennung des Produkt-Polynukleotids von dem Gerüst-Oligonukleotid.

9. Verfahren nach einem der Ansprüche 1 bis 8, wobei die Bedingungen für den Schritt des Ligierens eine Temperatur von mindesten 30 °C aufweisen.

10. Verfahren nach Anspruch 9, wobei die Bedingungen für den Schritt des Ligierens eine Temperatur von mindesten 42 °C aufweisen.

11. Verfahren nach Anspruch 10, wobei die Bedingungen für den Schritt des Ligierens eine Temperatur von mindesten 50 °C aufweisen.

12. Verfahren nach einem der Ansprüche 1 bis 11, wobei sich das Produkt-Polynukleotid um mindestens zehn Nukleotide über jedes Ende des Gerüst-Oligonukleotids hinaus erstreckt.

13. Verfahren nach Anspruch 12, wobei sich das Produkt-Polynukleotid um mindestens 25 Nukleotide über jedes Ende des Gerüst-Oligonukleotids hinaus erstreckt.

14. Verfahren nach Anspruch 13, wobei sich das Produkt-Polynukleotid um mindestens 75 Nukleotide über jedes Ende des Gerüst-Oligonukleotids hinaus erstreckt.

15. Verfahren nach einem der Ansprüche 1 bis 14, wobei das Gerüst-Oligonukleotid teilweise dupliziert wird auf das zentrale Oligonukleotid dupliziert wird, so dass eine einsträngige Region sowohl an dem ersten als auch an dem zweiten Ende des Gerüst-Oligonukleotids verbleibt bevor das Gerüst- und das zentrale Oligonukleotid mit den ersten und zweiten Oligonukleotiden in Kontakt gebracht werden.

16. Verfahren nach einem der Ansprüche 1 bis 15, wobei das Produkt-Polynukleotid markiert ist.

## Revendications

1. Procédé de construction d'au moins une espèce de polynucléotide produit à l'aide de populations de produits de synthèse d'oligonucléotide contenant une troncation, le procédé comprenant les étapes consistant à :
ligaturer ensemble un oligonucléotide central, au moins une espèce de premier oligonucléotide, et au moins une espèce de second oligonucléotide,
les oligonucléotides premier, central et second étant hybridés à un oligonucléotide d'échafaudage commun pendant la ligature,
l'oligonucléotide d'échafaudage étant complémentaire à la totalité de la longueur de l'oligonucléotide central, de sorte que le duplex formé soit stable dans les conditions de ligature, et l'oligonucléotide d'échafaudage étant complémentaire aux premier et second oligonucléotides uniquement sur un nombre limité de nucléotides, de sorte que les duplex formés ne soient pas stables dans les conditions de ligature, et dans lequel ledit oligonucléotide d'échafaudage est un sous-modèle en longueur.

2. Procédé selon la revendication 1, le procédé comprenant en outre les étapes consistant à :
duplexer partiellement un oligonucléotide d'échafaudage de longueur de sous-modèle ayant une première extrémité et une seconde extrémité avec un oligonucléotide central qui a une extrémité 5' et une extrémité 3', de sorte qu'une région à simple brin soit laissée aux première et seconde extrémités de l'oligonucléotide d'échafaudage ; et
ligaturer un premier et un second oligonucléotide, respectivement, aux extrémités 5' et 3' dudit oligonucléotide central en échantillonnant des première et seconde populations respectives de produits de synthèse d'oligonucléotide contenant une troncation avec les régions à simple brin aux extrémités dudit oligonucléotide d'échafaudage partiellement duplexé, dans lequel le premier oligonucléotide comprend une région parfaitement complémentaire en séquence à la région à simple brin au niveau de la première extrémité dudit oligonucléotide d'échafaudage et le second oligonucléotide comprend une région parfaitement complémentaire en séquence à la région à simple brin au niveau de la seconde extrémité dudit oligonucléotide d'échafaudage.

3. Procédé selon la revendication 2, dans lequel ledit duplexage partiel et ladite ligature sont effectués en une seule étape.

4. Procédé selon la revendication 2 ou 3, dans lequel chacune des régions à simple brin au niveau des extrémités de l'oligonucléotide d'échafaudage est inférieure ou égale à 10 nucléotides de long.

5. Procédé selon la revendication 4, dans lequel chacune des régions à simple brin au niveau des extrémités de l'oligonucléotide d'échafaudage est inférieure ou égale à 7 nucléotides de long.

6. Procédé selon la revendication 5, dans lequel chacune des régions à simple brin au niveau des extrémités de l'oligonucléotide d'échafaudage est inférieure ou égale à 5 nucléotides de long.

7. Procédé selon l'une quelconque des revendications 2 à 6, dans lequel ladite première population échantillonnée comprend les produits d'une première pluralité de synthèses d'oligonucléotide, les produits de synthèse de longueur totale d'au moins deux de ladite première pluralité de synthèses étant différents en séquence, et ladite seconde population comprend les produits d'une seconde pluralité de synthèses d'oligonucléotide, les produits de synthèse de longueur totale d'au moins deux de ladite seconde pluralité de synthèses étant différents en séquence.

8. Procédé selon l'une quelconque des revendications 1 à 7, comprenant en outre une étape consistant à séparer par tailles le polynucléotide produit de l'oligonucléotide d'échafaudage.

9. Procédé selon l'une quelconque des revendications 1 à 8, dans lequel les conditions de la ligature comprennent une température d'au moins 30 °C.

10. Procédé selon la revendication 9, dans lequel les conditions de la ligature comprennent une température d'au moins 42 °C.

11. Procédé selon la revendication 10, dans lequel les conditions de la ligature comprennent une température d'au moins 50°C.

12. Procédé selon l'une quelconque des revendications 1 à 11, dans lequel le polynucléotide produit s'étend sur au moins 10 nucléotides au-delà de chacune des extrémités de l'oligonucléotide d'échafaudage.

13. Procédé selon la revendication 12, dans lequel le polynucléotide produit s'étend sur au moins 25 nucléotides au-delà de chacune des extrémités de l'oligonucléotide d'échafaudage.

14. Procédé selon la revendication 13, dans lequel le polynucléotide produit s'étend sur au moins 75 nucléotides au-delà de chacune des extrémités de l'oligonucléotide d'échafaudage.

15. Procédé selon l'une quelconque des revendications 1 à 14, dans lequel l'oligonucléotide d'échafaudage est partiellement duplexé à l'oligonucléotide central, de sorte qu'une région à simple brin est laissée aux première et seconde extrémités de l'oligonucléotide d'échafaudage, avant de mettre en contact les oligonucleotides d'échafaudage et central avec les premier et second oligonucléotides.

16. Procédé selon l'une quelconque des revendications 1 à 15, dans lequel le polynucléotide produit est marqué.
